# EUROPEAN PATENT SPECIFICATION

(11) **EP 3 902 915 B1**
(45) Date of publication and mention of the grant of the patent: **23.08.2023**
(21) Application number: 19842751.0
(22) Date of filing: 20.12.2019
(51) Int. Cl.: C12N 15/11, C12N 15/90, C12N 15/65

(54) **METHOD FOR THE SELECTION OF CELLS BASED ON CRISPR/CAS-CONTROLLED INTEGRATION OF A DETECTABLE TAG TO A TARGET PROTEIN**
METHODE ZUR SELEKTION VON ZELLEN BASIEREND AUF CRISPR/CAS-GESTEUERTER INTEGRATION EINES DETEKTIERBAREN TAGS AN EIN ZIELPROTEIN
PROCÉDÉ DE SÉLECTION DES CELLULES EN FONCTION DE L'INTÉGRATION COMMANDÉE PAR CRISPR / CAS D'UN MARQUEUR DÉTECTABLE SUR UNE PROTÉINE CIBLE

(30) Priority: 30.12.2018 EP 18215918
(43) Date of publication of application: 03.11.2021
(73) Proprietor: F. Hoffmann-La Roche AG, 4070 Basel (CH)
(72) Inventor: HAAS, Alexander, 82377 Penzberg (DE)
(74) Representative: Skolaut, Alexander
(86) International application number: PCT/EP2019/086666
(87) International publication number: WO 2020/141109

(56) References cited:
- WO-A1-2018/148603
- WO-A2-2018/144087
- DEXUAN KUANG ET AL: "Tagging to endogenous genes of Plasmodium falciparum using CRISPR/Cas9", PARASITES & VECTORS, vol. 10, no. 1, 2 December 2017 (2017-12-02), pages 1-8, XP055600031, DOI: 10.1186/s13071-017-2539-0
- POORAN SINGH DEWARI ET AL: "An efficient and scalable pipeline for epitope tagging in mammalian stem cells using Cas9 ribonucleoprotein", ELIFE, vol. 7, 11 April 2018 (2018-04-11), XP055599579, DOI: 10.7554/eLife.35069
- BROCK ROBERTS ET AL: "Systematic gene tagging using CRISPR/Cas9 in human stem cells to illuminate cell organization", MOLECULAR BIOLOGY OF THE CELL, vol. 28, no. 21, 15 October 2017 (2017-10-15), pages 2854-2874, XP055538712, US ISSN: 1059-1524, DOI: 10.1091/mbc.e17-03-0209 cited in the application

## Description

The present invention lies in the technical field of non-therapeutic *(in vitro)* targeted integration of nucleic acids into cellular DNA in order to generate cells that express endogenous proteins in labelled form, and their application.

### Technological background

The CRISPR/Cas technology has gained a rapidly growing popularity among natural scientists in recent years (1). Although discovered in *Escherichia coli* back in 1987, the function of the CRISPR gene was not explained until much later, in 2007 (2,3). In 2012, the complete mechanism was decoded by Jennifer Doudna and Emmanuelle Charpentier and presented as a good alternative to existing genetic engineering tools (4). The first publication on CRISPR/Cas-based genome editing in human cells was disclosed in 2013 by Feng Zhang (5).

A common problem in researching new and/or unknown antigens is the lack of commercially available specific antibodies. This complicates characterization in terms of expression or cellular localization.

Normally, the antigen to be investigated has been provided with a tag and has been recombinantly produced in a cell. Because the expression occurs under the control of a viral promoter, leading to overexpression of the antigen, the experiments often yield distorted or incorrect data.

Kuang, D., et al., reported the tagging to endogenous genes of Plasmodium falciparum using CRISPR/Cas9 (Paras. Vect. 10 (2017) 1-8).

Dewari, P.S., et al., reported an efficient and scalable pipeline for epitope tagging in mammalian stem cells using Cas9 ribonucleoprotein (eLIFE 7 (2018) 35069).

### Summary

Object of the present invention is a new method for the provision of cells, which, for example, express a pharmacologically interesting cellular protein as a fusion protein with a marker protein, i.e. a proteinaceous detectable label. Such cells enable the study of this cellular protein as well as the metabolic processes in which this protein is involved directly within the cells or on the cell surface, i.e. in its natural environment. Thus, the labelled protein is expressed from its native gene locus under natural conditions, i.e., there is, for example, no overexpression or underexpression with respect to the protein's natural expression level, which is generally occurring in cases when the protein is recombinantly introduced as fusion protein with the marker into a cell.

The cells obtained by the method according to the present invention can be used, inter alia, in the identification and selection of antibodies that modify the biological activity of the endogenous target protein.

Thus, disclosed herein is a new method for studying pharmaceutically interesting proteins directly within cells or on cell surfaces, wherein the protein has been modified in the cell in its endogenous gene locus by fusion to a marker protein.

The invention described herein is based, at least in part, on the finding that by using CRISPR/Cas9 technology, it is possible to conjugate proteins directly within cells to a detectable label or tag directly at the protein's endogenous gene locus whereby native expression levels of the protein are/can be obtained in the modified cell.

The invention described herein is based, at least in part, on the finding that antibiotic-mediated double selection for Cas9 nuclease and the donor plasmid are most suitable for the knock-in of a marker protein (tag).

The invention described herein is based, at least in part, on the finding that significantly higher editing rates can be achieved using the donor plasmid in the circular conformation than when using the linearized form of the donor plasmid.

In the method according to the invention, a Cas9-encoding plasmid comprising a first resistance cassette (e.g., a puromycin resistance cassette) is co-transfected with a circular donor plasmid containing a second resistance cassette (e.g., a hygromycin B resistance cassette) as a repair template, as well as suitable synthetic crRNA and tracrRNA. The selection was a double selection for both resistances, e.g., with hygromycin B for the resistance to the donor plasmid and puromycin for the resistance to the Cas9 plasmid.

One aspect of the invention is an *(in vitro)* method for the provision/production of cells that express a fusion protein of a marker protein and a cell-endogenous target protein from the cell-endogenous gene locus of the target protein, wherein the method comprises the following steps:
a) transfecting the cells i) with a Cas9-encoding plasmid containing a nucleic acid that confers resistance to a first selection reagent, ii) with a circular donor plasmid comprising a) a first nucleic acid that confers resistance to a second selection reagent, and b) a second nucleic acid that encodes the marker protein and that is flanked each 3' and 5' by nucleic acids homologous to the integration site in the cell, wherein one of the flanking homologous nucleic acids is homologous to the terminal coding sequence of the target protein, iii) with a suitable crRNA, and iv) with a suitable tracrRNA,
b) culturing the cells in the presence of the first and the second selection reagent, and
c) selecting cells that perform cell division/growth under the conditions of step b), and thereby providing/producing cells that express a fusion protein of a marker protein and a cell-endogenous target protein.

Disclosed herein is an *(in vitro)* method for determining/characterizing whether an antibody is agonistic or antagonistic (or inactive) with respect to its target molecule, which comprises the following steps:
- optionally determining the biological activity of the target protein,
- incubating a cell that expresses a fusion protein of a marker protein and a cell-endogenous target protein, and that has been obtained by the method according to the current invention with the antibody to be tested,
- determining how the biological activity of the endogenous target protein is altered in the presence of the antibody to be tested, and characterizing the antibody as agonistic if the biological activity is increased upon incubation with the antibody and/or as antagonistic if the biological activity is reduced upon incubation with the antibody (and/or as inactive if the biological activity is not altered upon incubation with the antibody).

Disclosed herein is an *(in vitro)* method for the selection of an antibody that specifically binds to a target molecule, comprising the following steps:
- incubating a cell that expresses a fusion protein of a marker protein and a cell-endogenous target protein, and that has been obtained by the method according to the current invention with one or separately with two or more antibodies to be tested,
- determining whether the biological activity of the endogenous target protein is altered in the presence of the antibody to be tested and selecting the antibody if the biological activity is altered upon incubation with the antibody.

In a preferred embodiment, the cells are transfected simultaneously with all plasmids and nucleic acids.

In one embodiment, in step c), the cells that perform cell division and in which the fusion protein has been detected are selected.

In one preferred embodiment, steps b) and c) are the following steps:
b)
   1) culturing the cells in the presence of only the first selection reagent or in the presence of only the second selection reagent,
   2) selecting cells which divide/grow under the conditions of step 1),
   3) cultivating the cells selected in step 2) in the presence of the selection reagent not used in step 1),
c) selecting cells that perform cell division/growth under the conditions of step b) 3), thereby providing/producing cells that express a fusion protein of a marker protein and a cell-endogenous target protein.

In one embodiment, the first and the second selection reagent are selected from the group consisting of Neomycin/G418 (neomycin phosphotransferase), histidinol (histidinol dehydrogenase), hygromycin B (hygromycin phosphotransferase), xanthine-guanine phosphoribosyltransferase, thymidine kinase, adenine phosphoribosyl transferase, blasticidin, puromycin, zeocin, and mycophenolic acid. In one preferred embodiment, the first and second selection reagents are puromycin and hygromycin B or vice versa.

In one preferred embodiment, the final hygromycin B concentration is 50 µg/mL.

In one preferred embodiment, the final puromycin concentration is 2 µg/mL.

In one embodiment, the cell-endogenous target protein is a soluble protein, or a membrane-bound protein.

In one embodiment, in the presence of multiple possible insertion sites for the marker protein (tag) the nucleic acid encoding the marker protein is inserted at the site containing more and/or more specific gDNA binding sites.

In one preferred embodiment, the cell is a mammalian cell.

In one embodiment, the marker protein is introduced C-terminally or N-terminally to the endogenous target protein. In one preferred embodiment, the marker protein encoding nucleic acid is inserted N-terminally directly behind the start codon of the cell-endogenous protein.

In one embodiment,
i) the marker protein is inserted N-terminally to the target protein,
ii) the nucleic acid encoding the marker protein is inserted in the endogenous gene locus so that it is directly before (3' to) the first codon of the target protein in the mRNA of the fusion protein,
   and
iii) the 3'-flanking nucleic acid contains the start codon of the nucleic acid encoding the target protein or/and the 5'-flanking nucleic acid is homologous to the N-terminus of the target protein.

In one embodiment, a linker protein of at most 25 amino acids in length is inserted between the marker protein and the target protein. In one preferred embodiment, the linker protein comprises 4 to 10 amino acid residues.

In one embodiment, the marker protein encoding nucleic acid is inserted N-terminally directly behind the start codon of the cell-endogenous protein. In this embodiment, the 3' flanking nucleic acid contains the start codon of the nucleic acid encoding the target protein and the 5' flanking nucleic acid is homologous to the N-terminus of the target protein, or vice versa.

In one preferred embodiment, the nucleic acid encoding the marker protein does not contain a start codon.

In one embodiment, the marker protein is a fluorescent marker protein. In one embodiment, the fluorescent marker protein is selected from the group consisting of green fluorescent protein (GFP), TagBFP, mTagBFP, mTagBFP2, Azurite, EBFP2, mKalama1, Sirius, Sapphire (H9-40), T-Sapphire, ECFP, Cerulean, SCFP3A, mTurquoise (improved SCFP3A), mTurquoise2, monomeric Midoriishi-Cyan, TagCFP, mTFP1, EGFP, Emerald, Superfolder GFP, Monomeric Azami Green, TagGFP2, mUKG, mWasabi, Clover, mNeonGreen, EYFP, Citrine, Venus, SYFP2, TagYFP, monomeric Kusabira orange, mKOκ, mKO2, mOrange, mOrange2, mRaspberry, mCherry, mStrawberry, mTangerine, tdTomato, TagRFP, TagRFP-T, mApple, mRuby, mRuby2, and UnaG. In one embodiment, the fluorescent marker protein is GFP. In one preferred embodiment, the fluorescent marker protein is eGFP (enhanced green fluorescent protein).

In one embodiment, the marker protein is the Myc tag

In one preferred embodiment, the 3' and 5' flanking homologous nucleic acids have a length of about 1000 nucleotides (the 5' flanking homologous nucleic acid comprises about 1000 nucleotides of the sequence *upstream* of the insertion site and the 3' flanking homologous nucleic acid comprises about 1000 nucleotides of the sequence *downstream* of the insertion site).

### Description

### Definitions

CRISPR: Clustered Regularly Interspaced Short Palindromic Repeats; grouped short palindromic repeats at regular intervals
CAS Protein: CRISPR-associated-protein; has ribonuclease activity and can bind specific RNA sequences
CAS9: Endonuclease Cas9; binds the RNA sequence GUUUUAGAGCU(A/G)UG(C/U)UGUUUUG) (crRNA repeat) (SEQ ID NO: 34) and cuts DNA there
crRNA: consists of crRNA repeat sequence and crRNA spacer sequence; has a specific secondary structure; crRNA is bound by Cas9, thereby inducing conformational changes in Cas9 whereby target DNA can be bound by the crRNA spacer (complementary to target DNA); by exchanging the crRNA spacer sequence, target DNA can be altered (to target DNA complementary RNA sequence); crRNA repeat consists of 20 nucleotides; the 12 nucleotides adjacent to the PAM motif are crucial for binding specificity
PAM motif: protospacer adjacent motif; motif adjacent to the protospacer; Sequence NGG; in the target DNA; cutting of the target DNA takes place three nucleotides before the PAM
tracrRNA: trans-acting CRISPR RNA; partially complementary to the crRNA; forms an RNA double helix; activation by RNase III; binds target DNA; endonuclease function cuts near the binding site
sgRNA: single guide RNA; single RNA strand containing the crRNA and tracerRNA
Gene locus: Location of a gene on a chromosome; position of a gene in the genome; the gene location
endogenous: naturally occurring within a cell; naturally produced by a cell; endogenous gene locus/cell-endogenous gene locus: naturally occurring locus in a cell;
3' flanking sequence: sequence located at the 3' end (downstream of; below) a nucleotide sequence
5' flanking sequence: sequence located at the 5' end (upstream of, above) a nucleotide sequence
donor sequence: 5' flanking sequence - target sequence - 3' flanking sequence
donor plasmid: plasmid containing the donor sequence
flanking nucleotide sequence: sequence segment of a nucleic acid that precedes or follows the sequence to be inserted (= target sequence)

### CRISPR/Cas9 technology

The native CRISPR/Cas system is found in approximately 40% of bacteria and 90% of Archaea as part of the adaptive immune defense against viral invaders (1,6). CRISPR (Clustered Regularly Interspaced Short Palindromic Repeats) are short repeats of sequences that are periodically interrupted by specific spacer sequences. In the genome, this locus is flanked by CRISPR associated genes, Cas for short. These code for helicases and nucleases (1,7), among others.

The defense mechanism using CRISPR/Cas9 can be explained using the example of *Streptococcus pyogenes* as follows: A protospacer sequence of incoming foreign DNA is integrated into the native CRISPR locus. Translation of this region results in a pre-crRNA (pre-CRISPR RNA), which forms a complex through base pairing with the tracrRNA (trans-activating crRNA). This is further processed by, among others, RNase III, resulting in a crRNA:tracrRNA duplex acting as guide RNA (gRNA). The gRNA is complementary to the incoming foreign DNA, anneals and effects DNA cleavage by recruitment and activation of the Cas9 endonuclease. This has two nuclease domains, RuvC and HNH, each of which generates a break in both strands of DNA, resulting in a double strand break. The pre-requisite for this mechanism is that a short, conserved sequence, the PAM sequence (protospacer adjacent motif), is located below (downstream of) the gRNA target sequence (3,8). This is a binding signal for Cas9 and therefore essential for DNA cleavage. In the case of *S. pyogenes*, the Cas9 endonuclease cuts three bases upstream of the specific PAM 5'-NGG-3' sequence. Even with the fully complementary sequence homology of gRNA and a DNA locus, this is skipped by the nuclease in the absence of the PAM sequence. This enables the immune system to distinguish between its own and foreign DNA. Target sequences in the CRISPR locus of the bacterial genome do not contain a PAM sequence and are therefore protected from nuclease digestion (6,9).

In general, various CRISPR systems can be classified, which vary in their PAM sequence as well as in the number and types of Cas proteins involved. Type II from *S. pyogenes* is the best characterized and most frequently used in genetic engineering laboratories (8,10,11). The potential of this native system as a genetic engineering tool was recognized by Doudna and Charpentier in 2012. They also showed that the fusion of crRNA and tracrRNA into a single-guide RNA (sgRNA) generates DNA cleavages as efficiently as the native crRNA:tracrRNA duplex. The result is a molecular biology two-component system of sgRNA and Cas9 that can be used to generate genomic modifications quite easily (4,12).

The generation of DNA double-strand breaks leads to the activation of repair mechanisms in the cell. Non-homologous end-joining (NHEJ) is characterized as very error-prone, since it can lead to the formation of indel and frameshift mutations and as such, to the knock-out of the gene. Less frequently, repair takes place through homology directed repair (HDR). Thus, defined modifications of a locus can be carried out in the presence of a repair template, and even entire sequence blocks can be incorporated into a specific genome region (13, 14).

### Exemplary, specific embodiments of the invention

An often occurring problem in the study of new and/or unknown proteins, e.g. as antigens for therapeutic antibodies, is the lack of commercially available specific antibodies for said protein. This complicates the characterization of the protein, e.g. with respect to the expression or the cellular localization.

Hitherto, the protein/antigen to be examined has been conjugated ex-vivo to a marker protein, then re-introduced into the cell and recombinantly produced. Because the artificial, i.e. exogenous, fusion protein is recombinantly introduced into a cell, the expression takes place under the control of a viral promoter, which leads to overexpression of the fusion protein compared to its endogenous expression level. As a result, experiments based thereon often yield distorted or incorrect data.

The current invention is based, at least in part, on the finding that by means of CRISPR/Cas9 technology, it is possible to fuse a protein/antigen with a marker protein endogenously, i.e. to provide it with a detectable tag, whereby the fusion protein is expressed at the endogenous gene locus and whereby the native gene expression rates/levels are maintained.

This so called "knock-in" of sequences into the genomic DNA of a cell has proven to be non-trivial.

The current invention is based, at least in part, on the finding that antibiotic-mediated double selection on the first plasmid bearing the Cas9 nuclease and the second donor plasmid bearing the target sequence are best suitable for the knock-in of a marker protein. Although it was possible to obtain clones with correct knock-in with individual selection on the Cas9 nuclease, this was with significantly lower efficiency. The selection via an additionally introduced CD4 marker in combination with an antibiotic selection on the donor plasmid proved to be completely unsuccessful.

The invention described herein is based, at least in part, on the finding that, using the donor plasmid in the circular conformation, significantly higher incorporation rates/editing rates/efficiency can be achieved than when using the linearized form of the donor plasmid.

It should be noted that the method according to the current invention can be carried out with any endogenous gene of a target cell. The stronger the natural expression of this gene, the stronger is the detectable signal that can be achieved via the fused marker protein.

It should also be noted that the nature of the endogenous protein, i.e. whether it is soluble, membrane-associated or membranous, plays no role in the method according to the current invention.

The position at which the marker protein is introduced is also completely variable. It can be fused/introduced N-terminally, internally (only if, the biological function of the protein is not destroyed as a result) or C-terminally. The N-terminus and the C-terminus of the endogenous protein are nevertheless preferred for fusion.

It should also be noted that any detectable marker protein can be used. Fluorescent marker proteins are particularly preferred. However, conformational marker proteins can also be used. The detection thereof takes place via a labeled secondary antibody.

It should be noted that the method according to the current invention can be carried out with any cell. Mammalian cells are preferred. Human cells are more preferred.

### Example: Alpha Tubulin 1 beta (TUBA1B) as an endogenous gene

The endogenous α-tubulin 1β chain (TUBA1B) was selected as a non-limiting example of a protein/potential antigen. This protein was N-terminally tagged with an eGFP marker protein as a detectable label.

Alpha tubulin 1 beta (TUBA1B) is a subtype of alpha-tubulin and one of five tubulin isoforms, which are expressed differently depending on cell type, tissue, and stage of development (15).

The endogenous TUBA1B gene was N-terminally fused with an enhanced green fluorescent protein (eGFP) marker protein/tag/detectable label (SEQ ID NO: 32) in adherent growing HEK293A cells by means of the CRISPR/Cas9 technique. For this purpose, the eGFP coding sequence (SEQ ID NO: 33) was inserted into the genome of the cells so that it follows at the mRNA level directly behind the endogenousTUBA1B start codon.

When the target gene was analyzed at the genomic level, the start codon ATG was the last triplet of exon 1. This resulted in two possible insertion sites for the tag: directly behind the ATG at the 3' end of exon 1 or at the 5' end in front of exon 2. These two gene loci were examined for possible gRNA binding sites. It was found that clearly more specific gRNA binding sites are present near the 5' end of exon 2 than behind the ATG in exon 1.

Thus, in the presence of multiple possible insertion sites, in one embodiment of the method of the current invention, the nucleic acid encoding the marker protein is inserted at the site of the endogenous gene locus of the target protein in the cell that contains more and/or more specific gDNA binding sites.

On this basis, knock-in was performed in front of exon 2 using three specific gRNAs (Figure 1 and Figure 2). By recruiting the Cas9 nuclease, these generated a double strand break 23 to 80 bases away from the insertion site. Through co-transfection with a repair template, also called a donor plasmid or short donor, the eGFP tag was precisely incorporated by means of homologous recombination. For this purpose, the sequence to be incorporated, precisely the eGFP sequence without an internal ATG and with a C-terminal G4S linker flanked on each side by a 1 kb long homologous sequence (arm), was placed on the donor plasmid. The 5'-homologous sequence comprised about 1 kb of the upper/upstream sequence from the insertion site and the 3'-homologous sequence comprised about 1 kb of the lower/downstream sequence from the insertion site (Figure 3).

Three knock-in strategies were performed, whereby the Cas9 nuclease, a specific gRNA, and the donor plasmid were required for all strategies. The strategies differed in the composition used and the selection method.

In the Knock-in Strategy V1, a Cas9-encoding plasmid comprising a puromycin-resistance cassette was co-transfected with a circular donor plasmid without a selection marker as well as specific synthetic crRNA and tracrRNA. The selection was carried out exclusively with puromycin on the Cas9-encoding plasmid.

In the Knock-in Strategy V2, a Cas9-encoding plasmid comprising a puromycin-resistance cassette was co-transfected with a circular donor plasmid with a hygromycin B-resistance cassette as well as specific, synthetic crRNA and tracrRNA. The selection was carried out as double selection with hygromycin B for the donor plasmid and puromycin for the Cas9-encoding plasmid.

In the Knock-in Strategy V3, the Cas9 nuclease and the sgRNA were combined on a single plasmid. This additionally contained a CD4 marker for selection. The donor plasmid corresponded to that of the strategy V2. Thus, the double selection was carried out via hygromycin B for the donor plasmid, as well as for CD4 for the nuclease/gRNA plasmid.

For the V2 and V3 strategies, in addition two different approaches were used: with and without linearization of the donor plasmid prior to transfection.

A repair template was generated for the specific knock-in of the eGFP marker protein by means of a homologous recombination: in one case a donor plasmid without a selection marker and in the other case a donor plasmid with a hygromycin B-resistance cassette.

In addition to the origin of replication (ori), the donor plasmid without a selection marker contains an ampicillin-resistance cassette, the eGFP sequence to be introduced with homologous 3' and 5' flanking sequences. The 3'-homologous sequence was amplified by PCR from genomic DNA. After gel extraction, this was used as a template for the second PCR with primers specific for the 3'-homologous sequence. All PCR samples showed a clean band of the expected size in the agarose gel and were purified from the gel. PCR on genomic DNA from HEK293A wild-type cells was done with the TUBA1B specific primers D_5'HA fwd (SEQ ID NO: 12) and D_5'HA rev (SEQ ID NO: 13) for generating the 5'-homologous sequence. PCR on genomic DNA from HEK293A wild-type cells with TUBA1B specific primers D_3'HA-Z fwd (SEQ ID NO: 18) and D_3'HA-Z rev (SEQ ID NO: 19) was done for generating an intermediate product. Therefrom by means of PCR with the primers D_3'HA fwd (SEQ ID NO: 16) and D_3'HA rev (SEQ ID NO: 17) the 3'-homologous sequence with point-mutations for the gRNA1 and gRNA2 binding sites was generated. The four fragments, backbone, 5'HA, eGFP, and 3'HA (HA = homologous arm/sequence), had overlapping sequences at their ends, allowing simultaneous ligation to be performed in the correct order and orientation by means of seamless cloning. The correct cloning was confirmed by sequencing. The donor plasmid was generated from the final construct with a hygromycin B-resistance cassette. This served as a template for PCR to amplify the complete 5'HA-eGFP-3'HA fragment. The PCR product was directly purified and digested with restriction enzymes and ligated into the backbone of plasmid pAH-0163. It contains an ampicillin-resistance cassette and a hygromycin B-resistance cassette in addition to the ori. The digested samples were separated in agarose gel, excised and purified. Ligation in the correct orientation was accomplished via the complementary ends formed by restriction digestion. The correctness of the donor plasmid with the hygromycin B-resistance cassette was also verified by sequencing.

HEK293A wild-type cells were treated for 72 h with various concentrations of the antibiotics (a) hygromycin B and (b) puromycin, respectively. Cell viability was determined with the CellTiter-Glo assay.

At a concentration of 20 µg/mL, the cell viability of HEK293A (wild-type) cells decreased to less than 20%. At further increasing concentrations up to 300 µg/mL, the viability of the cells varied between 10% and 30%. For the selection, a final hygromycin B concentration of 50 µg/mL was used. At this concentration, a viability of about 12% is expected.

For puromycin, cell viability decreased slowly at the lowest concentrations and was below 15% at a concentration of 2 µg/mL puromycin. For the selection, a final puromycin concentration of 2 µg/mL was used, at which a viability of about 12% is to be expected.

### 1) Knock-in strategy V1: Single selection of Cas9-encoding plasmid by means of an antibiotic

Cas9-PuroR plasmid
Circular donor plasmid
crRNA1 or crRNA2
tracrRNA

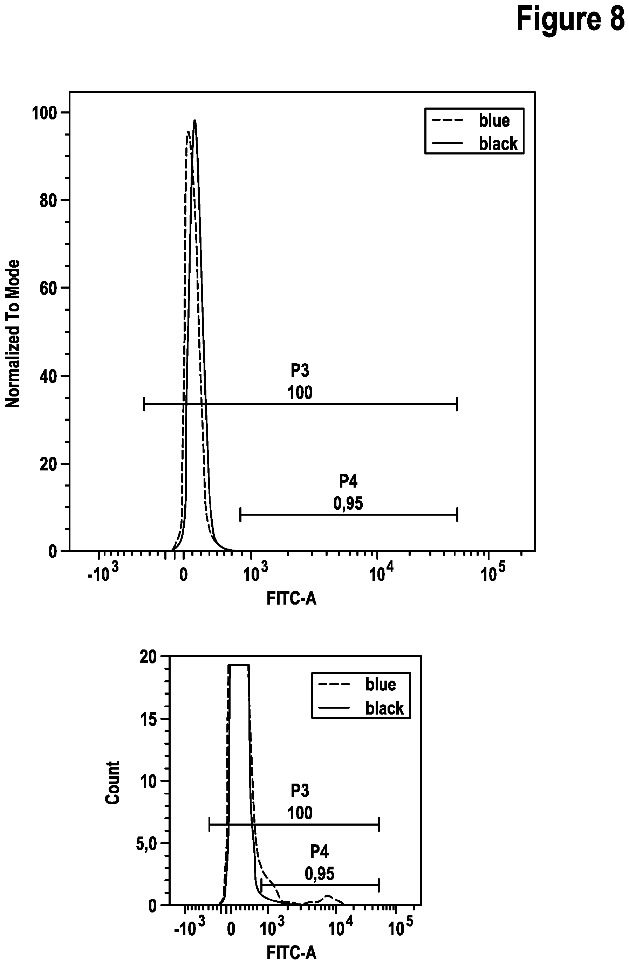

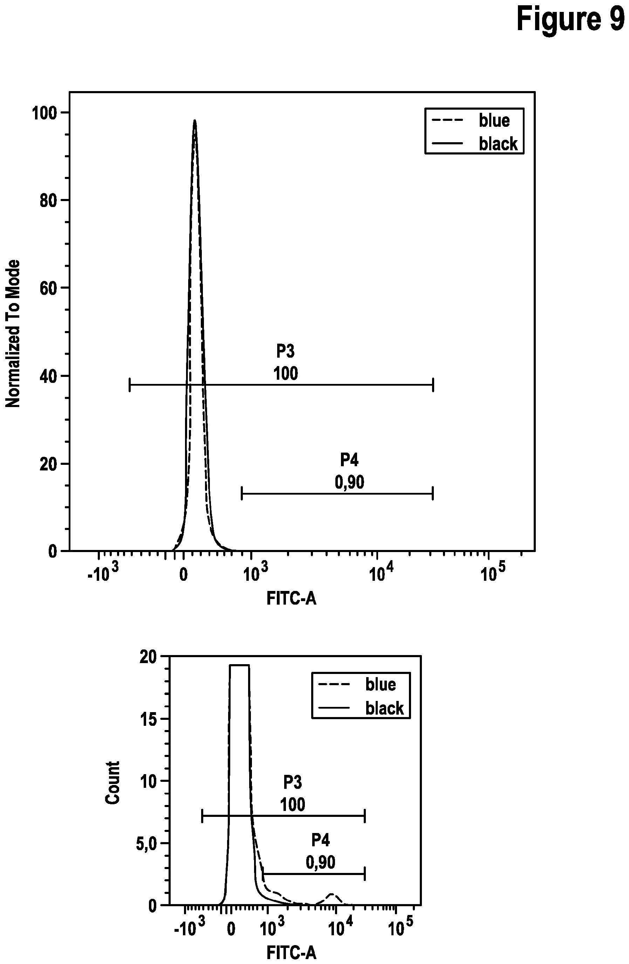

In the first knock-in strategy, the cells were transfected with the Cas9-PuroR plasmid, the circular donor plasmid, as well as specific, synthetic crRNA and tracrRNA. The selection was made exclusively on the Cas9 plasmid with puromycin for 10 days. Thereafter, single cells of the cells transfected with crRNA1 (SEQ ID NO: 07) or crRNA2 (SEQ ID NO: 08), respectively, were deposited in ten 96-well plates. For each crRNA, nine plates containing viable single cells from the gate P3 and one plate with FITC-positive, viable single cells from the gate P4 were deposited (Figures 8 and 9, the FITC signal of the samples (blue) is normalized and displayed (enlarged) together with the wild-type (black) in histogram). eGFP incorporated in the cells can be detected via the FITC channel by FACS (fluorescence-activated cell sorting). In the magnified view, the histograms of the FITC signal showed that a small portion of the cell population indicated a change in the FITC signal (Figures 8 and 9). The increased FITC signal can be attributed to the expression of eGFP, which is only possible if the eGFP sequence from the donor plasmid has been introduced in-frame into a translated region in the genome, since the start codon is missing.

The growth rate of the deposited single cells was determined microscopically after 3 weeks, and totaled 63%. Subsequently, the cells by FACS were analyzed for their FITC signal (results in Table 1).

**Table 1: For each crRNA and each gate, the number of potentially positive clones was identified after single-cell deposition. The total number of examined clones is stated in parentheses. The efficiency resulted from the ratio of the number of potentially positive clones to the number of clones examined.**

| | **Number of potentially positive clones** | | **Efficiency** | |
|---|---|---|---|---|
| | crRNA1 | crRNA2 | crRNA1 | crRNA2 |
| Plates from gate P3 | 2 (452) | 1 (399) | 0.44% | 0.25% |
| Plates from gate P4 | 11 (68) | 9 (64) | 16.1% | 14.7% |
| total | **23 (983)** | | **2.3%** | |

Clones for which the FITC signal was significantly higher than that of the wild-type were further expanded and characterized. A total of 23 potentially positive clones were detected from a total of 983 analyzed clones (see Table 1). In the first FACS screen, all clones indicated a shift into the FITC-positive region of about one log step.

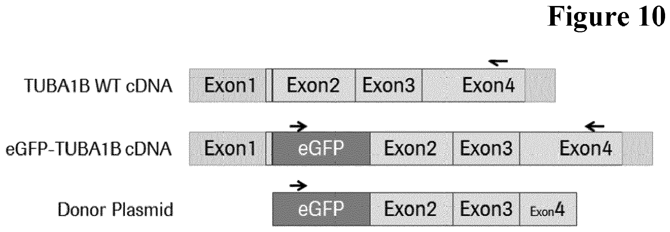

The clones were regularly analyzed by means of FACS screening to check their signal stability during continuous cultivation. The FACS data after 17 passages of cultivation showed a few differences between the clones. Many clones had a signal comparable to that of the first FACS screening. Two clones showed a significant loss in their FITC signal strength (Figure 10, Table 2).

**Table 2: Analysis of the grown clones by FACS for an eGFP signal. Gating was done on viable single cells via the FSC and SSC channels. It was possible to detect the presence of eGFP via the FITC channel. Clones with a shift into the FITC-positive region were expanded and further analyzed. The stability of the FITC signal was checked again at passage 17 after continuous cultivation. ++ = no change; + = slight reduction in the signal; o = strong reduction in the signal; - = no signal.**

| **Clone** | **Stability** |
|---|---|
| V1 g1 P6 E3 | ++ |
| V1 g1 P7 E7 | ++ |
| V1 g1 P10 A12 | o |
| V1 g1 P10 B5 | o |
| V1 g1 P10 B6 | + |
| V1 g1 P10 E5 | + |
| V1 g1 P10 B11 | + |
| V1 g1 P10 F12 | ++ |
| V1 g1 P10 D6 | + |
| V1 g1 P10 G7 | ++ |
| V1 g1 P10 E1 | + |
| V1 g1 P10 G11 | + |
| V1 g1 P10 H10 | ++ |
| V1 g2 P10 B6 | + |
| V1 g2 P10 F6 | - |
| V1 g2 P10 G6 | ++ |
| V1 g2 P8 A3 | ++ |
| V1 g2 P10 F5 | + |
| V1 g2 P10 B1 | + |
| V1 g2 P10 G10 | + |
| V1 g2 P10 B2 | o |
| V1 g2 P10 G11 | + |
| V1 g2 P10 E5 | + |

The FACS data show that many of the identified clones have a stable eGFP signal.

The knock-in was verified at the genomic level. For this purpose, a PCR was performed on the cDNA of the clones. The total RNA was isolated from the cells and converted to cDNA. The PCR was performed with a forward primer (SEQ ID NO: 30), which binds in the eGFP region, and a reverse primer (SEQ ID NO: 31) that binds in exon 4 of TUBA1B. The primer were chosen so that a specific product could be amplified only when a precise introduction of the eGFP sequence before the exon 2 of TUBA1B had occurred. In the wild-type cells, only the reverse primer could bind. Only the forward primer could bind to the donor plasmid, because the binding site of the reverse primer lies outside the sequence of the 3'-homologous sequence. Therefore, even with a possible random integration of the plasmid into the genome of the cells, no amplification product should arise (Figure 10).

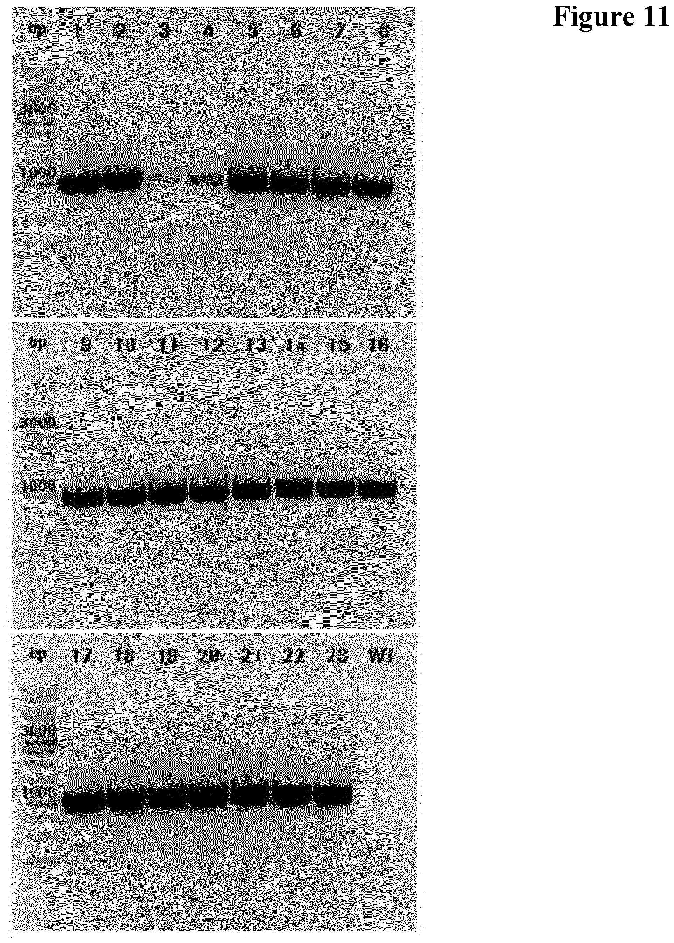

The PCR gave clean bands in the agarose gel without any noticeable by-products (Figure 11). The knock-in was verified at the DNA level by PCR and sequencing (data not shown). The bands were excised, purified and sequenced. As a result, the three sequence differences present in the DNA of all clones are silent mutations that do not cause any change in the protein sequence.

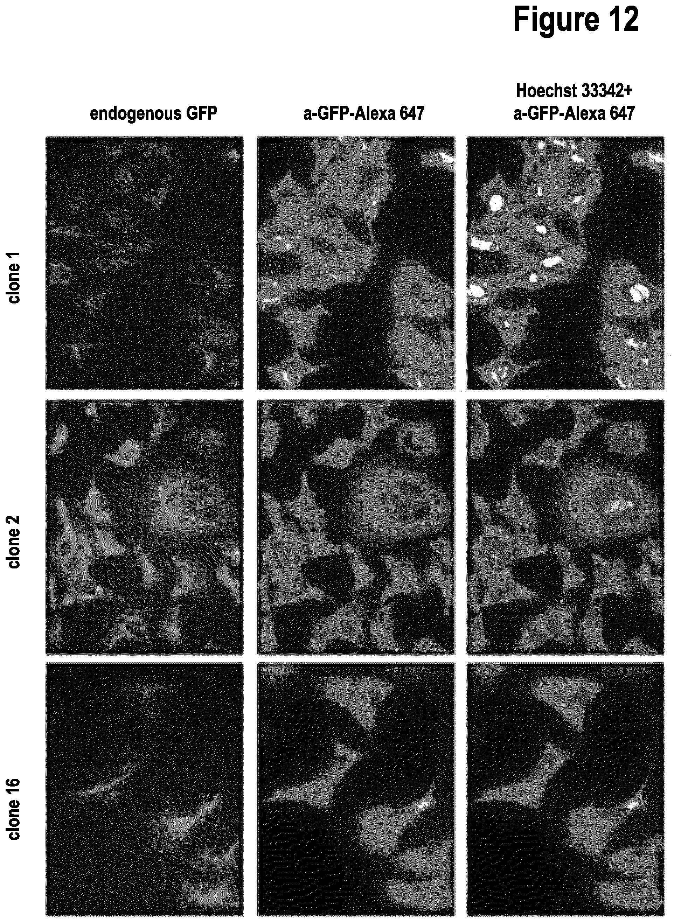

The functionality of the protein, i.e., the ability of the eGFP-TUBA1B fusion protein to form beta-tubulin heterodimers and microtubules therefrom, was microscopically verified. For this, the cells were fixed, permeabilized and stained with the Alexa Fluor 647 conjugate anti-GFP antibody. In addition, the cell nuclei were stained with Hoechst 33342. The confocal microscopic images were taken in the Operetta. Figure 12 shows representative images of clones 1, 2 and 16.

For all clones, the endogenous eGFP signal was not sufficient for microscopy, so additional staining of the eGFP with the Alexa Fluor 647 conjugate anti-GFP antibody was performed. This staining revealed clearly recognizable structures of the tubulin cytoskeleton, thus revealing filaments and some cellular protrusions (Figure 12).

In clones 3 and 4, some cells showed a weak GFP signal with the help of antibody staining, although cells without detectable GFP were also found. In general, clones 3 and 4 showed unclear results in all experiments. In the sequencing, it was possible to identify diverse mixed sequences. In addition, microscopic analysis showed that eGFP-TUBA1B was only present in part of the cells. This suggested that both clones are mixed clones.

The cells of clone 23 did not show any GFP signal in the microscope, neither endogenously nor with staining by the GFP antibody. It was not possible to detect eGFP-TUBA1B fusion protein in the cells of clone 23. However, a correct insertion of the eGFP sequence was detected several times at the DNA level. This suggested that the gene had been shut down in the cells. The knock-in had apparently been achieved correctly in this clone. However, it was unusable without expression of the modified protein.

### Summary of the Knock-in Strategy V1:

The Knock-in Strategy V1 is based on single selection with puromycin on the Cas9 nuclease plasmid. After the single-cell deposition, it was possible to identify 23 clones, which were characterized by means of FACS, imaging analyses, PCR and sequencing. With these analyses, it could be shown that in 20 clones knock-in occurred at the genomic level. The desired eGFP-TUBA1B fusion protein was expressed in the cells and showed no significant loss of function. The properties of the 23 clones are summarized in Table 3.

**Table 3: List of all the clones from the Knock-in Strategy V1 with summarized results.**

| **No** • | **Clone** | **% FITC-positive first screen** | **% FITC-positive last screen** | **Western Blot eGFP-TUBA1B** | **Imaging** | **Sequence cDNA** | **Posit ive clon e** |
|---|---|---|---|---|---|---|---|
| 1 | g1 P6 E3 | 94.9 | 95 | Positive | Positive | Variant A | Yes |
| 2 | g1 P7 E7 | 91.6 | 93.8 | Positive | Positive | Variant B | Yes |
| 3 | g1 P10 A12 | 63.2 | 35.1 | Negative | Partially | mixed sequence s | No |
| 4 | g1 P10 B5 | 61.2 | 37.2 | Negative | Partially | mixed sequence s | No |
| 5 | g1 P10 B6 | 99.8 | 97.7 | Positive | N/A | Variant A | Yes |
| 6 | g1 P10 B11 | 86.6 | 92.8 | Positive | N/A | Variant A | Yes |
| 7 | g1 P10 D6 | 96.9 | 89.9 | Positive | N/A | Variant A | Yes |
| 8 | g1 P10 E1 | 84.6 | 91.8 | Positive | N/A | Variant A | Yes |
| 9 | g1 P10 E5 | 98 | 84.3 | Positive | N/A | Variant A | Yes |
| 10 | g1 P10 F12 | 93.1 | 96 | Positive | N/A | Variant B | Yes |
| 11 | g1 P10 G7 | 97.3 | 96.8 | Positive | N/A | Variant A | Yes |
| 12 | g1 P10 G11 | 94.7 | 92.5 | Positive | N/A | Variant A | Yes |
| 13 | g1 P10 H10 | 90.4 | 94.7 | Positive | N/A | Variant B | Yes |
| 14 | g2 P8 A3 | 96.5 | 97 | Positive | N/A | Variant B | Yes |
| 15 | g2 P10 B1 | 95.8 | 87.9 | Positive | N/A | Variant A | Yes |
| 16 | g2 P10 B2 | 68.6 | 50.4 | Positive | Positive | Variant C | Yes |
| 17 | g2 P10 B6 | 99.2 | 94.8 | Positive | N/A | Variant A | Yes |
| 18 | g2 P10 F5 | 94.2 | 90.2 | Positive | N/A | Variant A | Yes |
| 19 | g2 P10 G6 | 57.8 | 64.4 | Positive | Positive | Variant C | Yes |
| 20 | g2 P10 G10 | 96.5 | 87.2 | Positive | N/A | Variant A | Yes |
| 21 | g2 P10 G11 | 96.8 | 85.2 | Positive | N/A | Variant A | Yes |
| 22 | g2 P10 E5 | 93.9 | 86.5 | Positive | N/A | Variant A | Yes |
| 23 | g2 P10 F6 | 95.1 | 1.1 | Negative | Negative | Variant A | No |

### 2) Knock-in Strategy V2: Double selection on Cas9 and donor plasmids by means of antibiotics

Cas9-PuroR plasmid
Circular and linearized donor plasmid with hygromycin B-resistance cassette crRNA1 or crRNA2 or crRNA3
tracrRNA

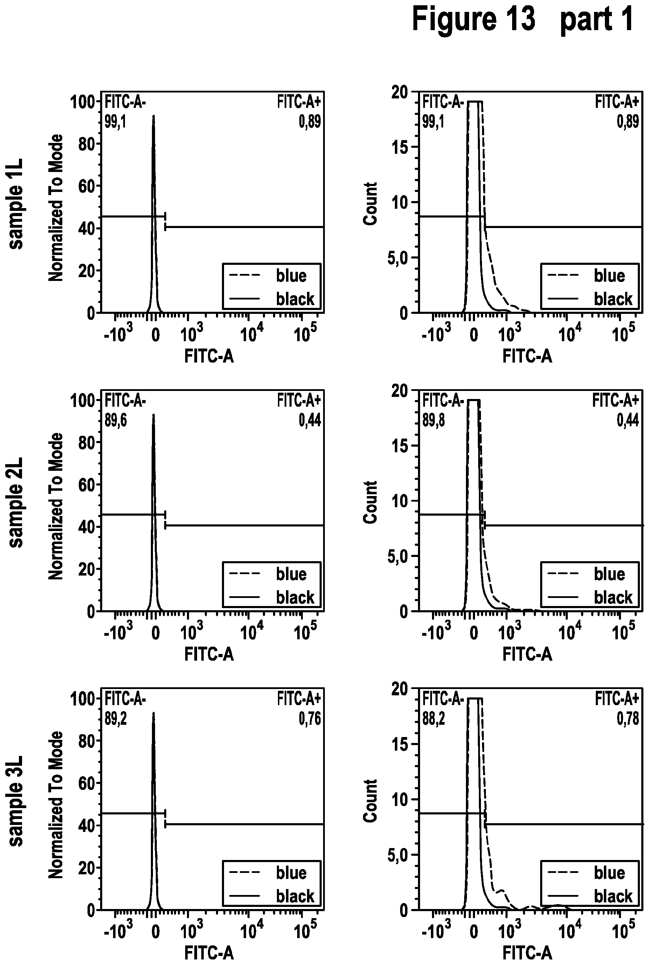

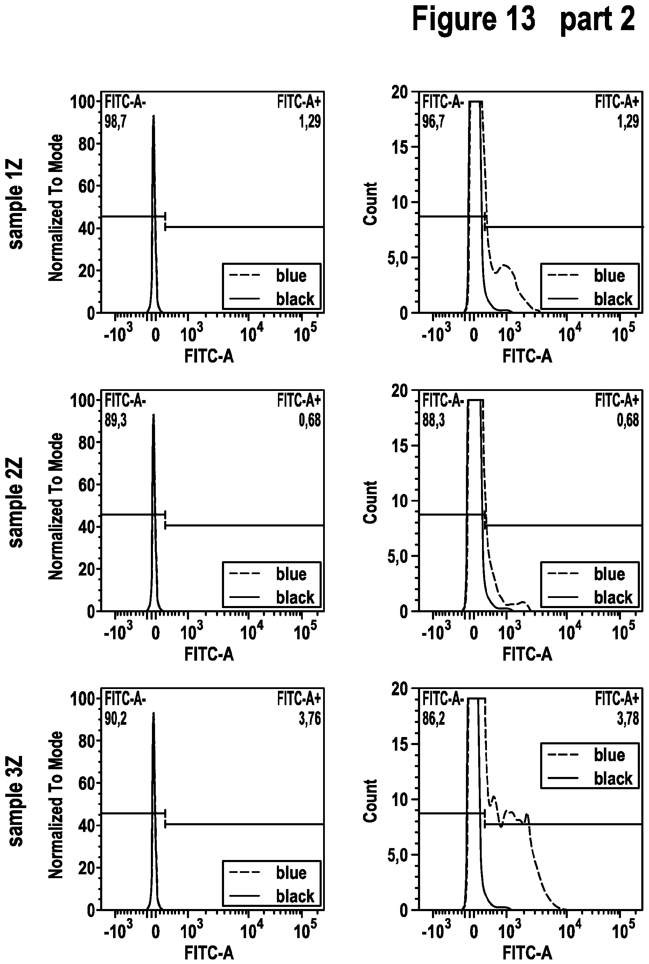

In the Knock-in Strategy V2, the cells were transfected with the identical crRNAs and tracrRNAs, as well as the Cas9-PuroR plasmid as in the Knock-in Strategy V1. Each of the three crRNAs (SEQ ID NO: 07-09) was combined with the linearized or circular donor plasmid with the hygromycin B-resistance cassette. Selection was performed via puromycin for the Cas9 plasmid and hygromycin B for the donor plasmid for a total of 10 days. Four 96-well plates with FITC-positive, viable single cells were subsequently deposited from each transfection. Differences in the FITC signal between the wild-type and the transfected cells were evident in the enlarged view of the histograms. The proportion of FITC-positive cells was between 0.4% and 3.8%. When comparing the samples from the linear and the circular donor plasmids, it was found that the proportion of FITC-positive cells in the transfection with the circular donor plasmid was higher for all three crRNAs than in the transfection with the linearized donor plasmid. The most intense signal was found in the sample of crRNA3 and the circular donor plasmid (Figure 13).

After a three-week incubation in the incubator, the growth rate of the deposited single cells was determined microscopically. It averaged 34.3%. All grown clones were analyzed by FACS for their FITC signal. An eGFP expression in the cells leads to the detection of an increased FITC signal compared to the wild-type, i.e. non-transfected cells. A total of 118 potentially positive clones were identified from a total of 496 analyzed clones. This corresponded to an overall efficiency of 24.3%. The proportion of potentially positive clones among the samples containing the circular donor plasmid was 33.3%, more than twice as high as that for the linearized donor plasmid at 15.3% (Table 4).

**Table 4: Identification of potentially positive clones from Knock-in Strategy V2 using FACS after single-cell deposition. The number of potentially positive clones identified after single cell deposition is given for each crRNA with linear or circular donor plasmid. The total number of examined clones is stated in parentheses. The efficiency resulted from the ratio of the number of potentially positive clones to the number of clones examined.**

| | **Number of potential clones** | | **Efficiency** | |
|---|---|---|---|---|
| | Linear donor plasmid | Circular donor plasmid | Linear donor plasmid | Circular donor plasmid |
| crRNA1 | 7 (97) | 40 (75) | 7.2% | 53.3% |
| crRNA2 | 0 (81) | 1 (98) | 0% | 1% |
| crRNA3 | 33 (84) | 37 (61) | 39.3% | 60.7% |
| total | 40 (262) | 78 (234) | 15.3% | 33.3% |
| | **118 (496)** | | **24.3%** | |

Of the 118 potentially positive clones, 40 clones were randomly selected and expanded for further validation. During continuous cultivation, the stability of the FITC signal was regularly checked by FACS. In the screening after 12 passages of cultivation, 32 of the 40 clones examined showed an increased FITC signal as compared to the wild-type. For clones V2 3Z P1 B5 and V2 3Z P2 D10, for example, this was comparable to that from the first FACS screening. Some clones showed a reduction in the FITC signal during continuous cultivation as compared to the first FACS screening. Nevertheless, it was higher in comparison to the wild-type. Only for three clones there was a failure to detect any FITC signal after 12 passages.

**Table 5: Analysis of the grown clones by FACS for an eGFP signal. Gating was done on viable single cells via the FSC and SSC channels. It was possible to detect the presence of eGFP via the FITC channel. Clones with a shift into the FITC-positive region were expanded and further analyzed. The stability of the FITC signal was checked again after continuous cultivation at passage 12. ++ = no change; + = slight reduction in the signal; o = strong reduction in the signal; - = no signal.**

| **Clone** | **Stability** |
|---|---|
| V2 1L P2 F5 | + |
| V2 3L P3 D9 | + |
| V2 1Z P1 F6 | + |
| V2 3Z P1 B5 | ++ |
| V2 3Z P2 D10 | ++ |
| V2 1Z P1 F3 | ++ |
| V2 1Z P1 E4 | + |
| V2 1Z P2 D7 | + |
| V2 1Z P2 F10 | + |
| V2 1Z P3 C6 | + |
| V2 1Z P3 D10 | + |
| V2 1Z P3 E11 | + |
| V2 1Z P2 E8 | + |
| V2 1Z P2 F7 | + |
| V2 3L P4 G6 | - |
| V2 1Z P1 D5 | ++ |
| V2 1Z P4 D5 | o |
| V2 1Z P4 E2 | + |
| V2 3Z P1 F8 | ++ |
| V2 3Z P2 C3 | ++ |
| V2 3Z P2 C8 | ++ |
| V2 3Z P3 C8 | ++ |
| V2 2Z P3 E5 | - |
| V2 3Z P1 E4 | ++ |
| V2 3Z P3 D3 | + |
| V2 1Z P1 E3 | ++ |
| V2 3L P2 C6 | - |
| V2 3L P2 C8 | o |
| V2 1Z P1 B4 | ++ |
| V2 1Z P1 C5 | ++ |
| V2 1Z P1 C7 | + |
| V2 1Z P3 F8 | + |
| V2 3L P2 E9 | o |
| V2 3Z P3 D10 | ++ |
| V2 3L P4 D9 | + |
| V2 1Z P4 C3 | ++ |
| V2 1Z P2 F9 | + |
| V2 1Z P4 F4 | - |
| V2 1Z P4 D9 | ++ |
| V2 3Z P1 B7 | ++ |

In addition to the FACS analysis, the 40 clones were further characterized by means of imaging, as well as PCR analysis and sequencing.

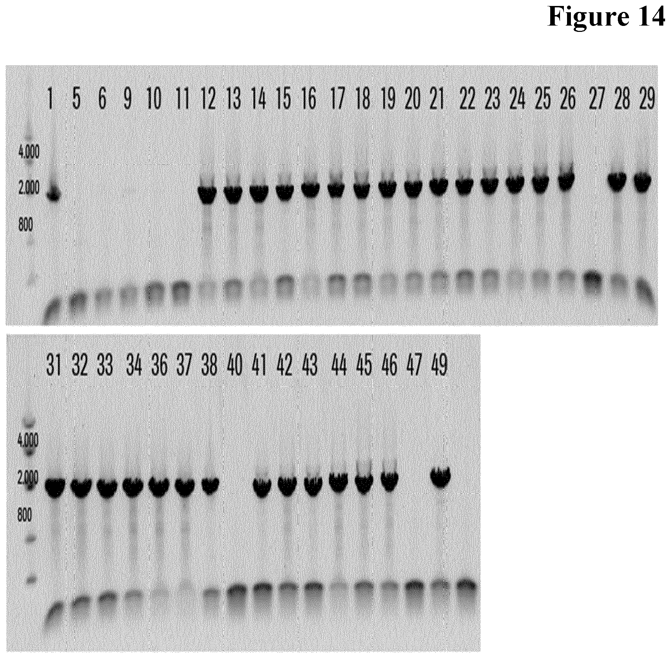

To confirm the knock-in at the genomic level, mRNA was isolated from the cells of the clones and transcribed into cDNA. On the cDNA, a PCR was set up with which the complete coding sequence of eGFP-TUBA1B could be amplified. It was only possible for both primers to bind when the insertion of the eGFP sequence was in front of TUBA1B (Figure 10). The PCR was analyzed on agarose gel and showed clean bands of the expected size. As expected, no product was produced in the wild-type test since only the reverse primer was able to bind (Figure 14).

The bands were excised, purified and sequenced. The two detected sequence differences in the DNA are silent mutations that do not cause any change in the protein sequence. Both sequence variants corresponded to the expected protein. The TUBA1B part was identical to that of the wild-type.

It was possible to determine a correct DNA sequence by PCR and sequencing for three negative clones. The clone 1L P2 F5, for example, showed positive results in all analyses, but only about half of the cells indicated positive signals by microscopy.

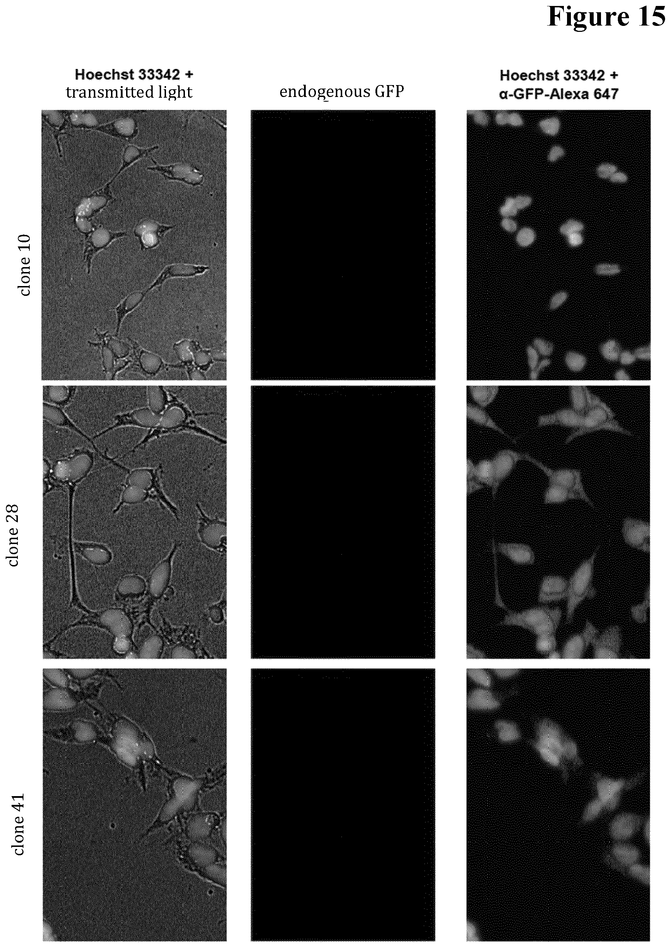

The functionality of the eGFP-TUBA1B fusion protein was analyzed microscopically. An investigation was carried out using the Operetta system to see whether the tagged protein continues to participate in the formation of microtubules in the clones, thus contributing to the maintenance of cell functionality. For this, the cells were fixed, permeabilized and stained with an Alexa Fluor 647 conjugate anti-GFP antibody. In addition, the cell nuclei were stained with Hoechst 33342. For example, clone 10 did not indicate any signal, even with staining using the anti-GFP antibody. In clone 41, the signals with the anti-GFP antibody were weak and very diffuse. Clone 28, on the other hand, showed clear signals from staining with the antibody in all cells. The cellular protrusions show good signals (Figure 15).

### Summary for Knock-in Strategy V2:

In the Knock-in Strategy V2, the cells were transfected with crRNA and tracrRNA, the Cas9-PuroR plasmid and the donor plasmid with a hygromycin B-resistance cassette. Each of the three crRNAs was used in combination with the respective linearized or circular donor plasmid. Selection took place via puromycin for the Cas9 plasmid and hygromycin B for the donor plasmid. Of 496 screened clones, 118 positive clones were identified. For further analysis, 40 clones were randomly selected and expanded. Results from FACS analysis, sequencing and imaging revealed that a precise eGFP knock-in was achieved in 29 clones.

The properties of the 40 selected clones are summarized in Table 6.

**Table 6: List of all the clones from the Knock-in Strategy V2 with the summarized results.**

| **No .** | **Clone** | **% FITC-positive first screen** | **% FITC-positive last screen** | **Western Blot eGFP-TUBA1B** | **Imaging** | **Sequence cDNA** | **Positive clone** |
|---|---|---|---|---|---|---|---|
| 1 | 1L P2 F5 | 50.8 | 56.7 | Positive | Partially | Variant A | Yes |
| 5 | 3L P2 C6 | 56.1 | 3.5 | Negative | N/A | Negative | No |
| 6 | 3L P2 C8 | 28.5 | 5 | Negative | N/A | Negative | No |
| 9 | 3L P2 E9 | 54.4 | 4 | Negative | N/A | Negative | No |
| 10 | 3L P3 D9 | 37.3 | 4 | Negative | Negative | Negative | No |
| 11 | 3L P4 G6 | 36.4 | 0.1 | Negative | N/A | Negative | No |
| 12 | 1Z P1 B4 | 63.9 | 75.3 | Positive | N/A | Variant B | Yes |
| 13 | 1Z P1 C5 | 42.6 | 68.5 | Positive | N/A | Variant B | Yes |
| 14 | 1Z P1 C7 | 47.5 | 29.1 | Positive | N/A | Variant B | Yes |
| 15 | 1Z P1 D5 | 45.5 | 35 | Positive | N/A | Variant B | Yes |
| 16 | 1Z P1 E4 | 61.1 | 37.3 | Positive | N/A | Variant B | Yes |
| 17 | 1Z P1 F6 | 93.4 | 50.1 | Positive | N/A | Variant B | Yes |
| 18 | 1Z P2 D7 | 83.1 | 43.7 | Positive | N/A | Variant B | Yes |
| 19 | 1Z P2 E8 | 54.3 | 39.6 | Positive | N/A | Variant B | Yes |
| 20 | 1Z P2 F7 | 73.5 | 38.3 | Positive | N/A | Variant B | Yes |
| 21 | 1Z P2 F10 | 72.6 | 46.8 | Positive | N/A | Variant B | Yes |
| 22 | 1Z P3 C6 | 70 | 69.6 | Positive | N/A | Variant B | Yes |
| 23 | 1Z P3 D10 | 94.9 | 54.6 | Positive | N/A | Variant B | Yes |
| 24 | 1Z P3 E11 | 74.1 | 21.4 | Positive | N/A | Variant B | Yes |
| 25 | 1Z P4 D5 | 76.7 | 26.1 | Positive | N/A | Variant B | Yes |
| 26 | 1Z P4 E2 | 47.8 | 26.5 | Positive | N/A | Variant B | Yes |
| 27 | 2Z P3 E5 | 21 | 0.9 | Negative | N/A | Negative | No |
| 28 | 3Z P1 B5 | 83.3 | 93.5 | Positive | Positive | Variant A | Yes |
| 29 | 3Z P1 E4 | 88.5 | 94.7 | Positive | N/A | Variant A | Yes |
| 31 | 3Z P1 F8 | 58.4 | 87.3 | Positive | Positive | Variant A | Yes |
| 32 | 3Z P2 C3 | 85.6 | 92.5 | Positive | N/A | Variant A | Yes |
| 33 | 3Z P2 C8 | 89.6 | 96.3 | Positive | N/A | Variant A | Yes |
| 34 | 3Z P2 D10 | 92.7 | 95.5 | Positive | Positive | Variant A | Yes |
| 36 | 3Z P3 C8 | 78.4 | 89.3 | Positive | N/A | Variant A | Yes |
| 37 | 3Z P3 D3 | 90.8 | 88.4 | Positive | N/A | Variant A | Yes |
| 38 | 3Z P3 D10 | 85 | 88.2 | Positive | N/A | Variant A | Yes |
| 40 | 3L P4 D9 | 23.3 | 7.6 | Negative | N/A | Negative | No |
| 41 | 1Z P1 F3 | 26.3 | 17.6 | Weak | Barely | Variant B | No |
| 42 | 1Z P2 F9 | 68.5 | 37.8 | Positive | N/A | Variant B | Yes |
| 43 | 1Z P3 E3 | 32.8 | 54.9 | Positive | N/A | Variant B | Yes |
| 44 | 1Z P3 F8 | 55.7 | 46.2 | Positive | N/A | Variant B | Yes |
| 45 | 1Z P4 C3 | 71.8 | 64.4 | Weak | Barely | Variant B | No |
| 46 | 1Z P4 D9 | 35.1 | 60.3 | Positive | N/A | Variant B | Yes |
| 47 | 1Z P4 F4 | 33.6 | 0.3 | N/A | N/A | Negative | No |
| 49 | 3Z P1 B7 | 72.7 | 90.1 | Positive | N/A | Variant A | Yes |

### 3) Knock-in Strategy V3: Double selection on Cas9 and donor plasmids using antibiotic and CD4 markers

pCas-Guide-EF1α-CD4 plasmid
Circular and linearized donor plasmid with hygromycin B-resistance cassette crRNA1 or crRNA2 or crRNA3
tracrRNA

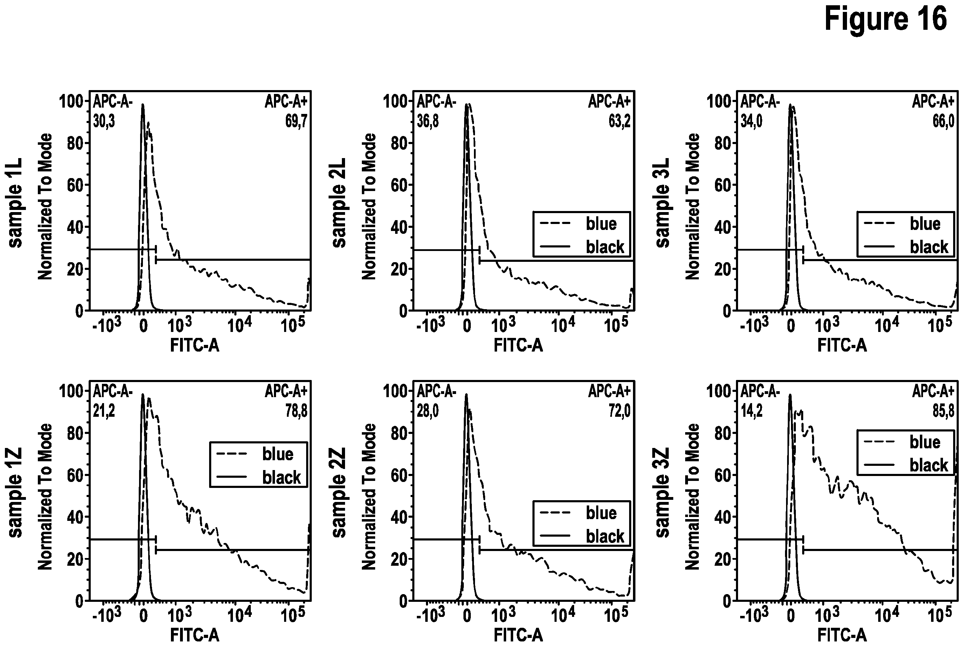

In the Knock-in Strategy V3, the cells were transfected with the circular or linear donor plasmid and a pCas-Guide EF1α-CD4 plasmid. This plasmid, referred to as "all-in-one", encodes the Cas9 nuclease, a gRNA and CD4. The selection was made in this strategy via hygromycin B for the donor plasmid, and with CD4 for the Cas9 nuclease and the gRNA. Three different pCas-Guide-EF1α-CD4 plasmids were used, each coding for one of the three gRNAs. This resulted in six transfections for this approach: three different gRNAs each in combination with the circular or the linearized donor plasmid. About 48 hours after transfection, the cells were stained with an APC-conjugated anti-CD4 antibody. HEK293A wild-type cells natively express no CD4, so these cells defined the APC negative gate. Only cells that had taken up the pCas-Guide-EF1α-CD4 plasmid expressed CD4 on the cell surface, which was detectable by FACS via the elevated APC signal after staining with the antibody. The samples all indicated a significantly increased APC signal. The transfected cells are between 63% and 86% in the APC positive gate. Comparing the samples with linear and circular donor plasmid from each gRNA, it was possible to see that proportionally more APC and, thus, CD4 positive cells were detected in the samples with the circular donor plasmid. From each transfection approximately 5×10⁵ CD4 positive cells were deposited as a cell pool using FACS sorting (Figure 16).

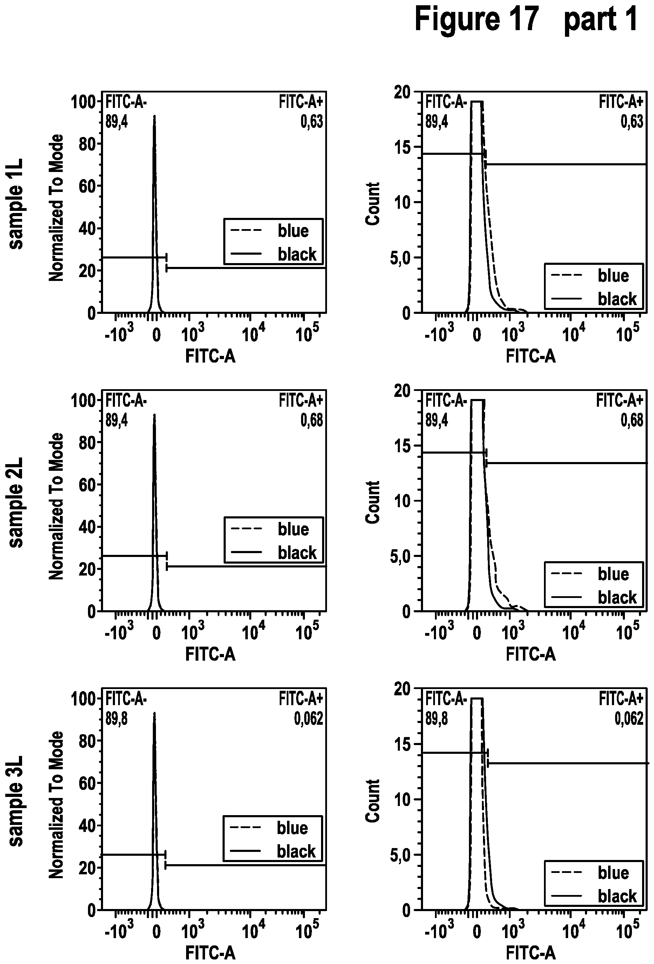

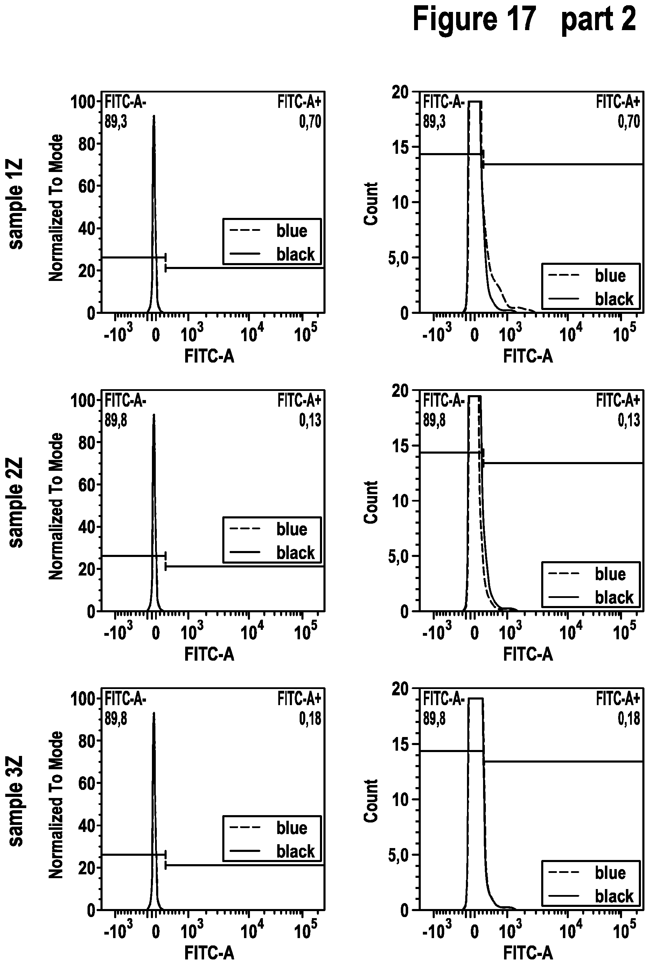

The cells were cultured after pool sorting for 24 h in 6-well plates. Next, selection with hygromycin B was performed for the donor plasmid for 10 days. Subsequently, single cells from each transfection were deposited in four 96-well plates. Differences in the FITC signal between the wild-type and the transfected samples were evident in the enlarged view of the histograms. Samples 1L, 1Z and 2L only showed very weak FITC-positive signals, well below 1%. Samples 2Z and 3Z showed no difference in FITC signal compared to the wild-type. Sample 3L showed even weaker FITC signals than the wild-type. Gating on FITC-positive cells was therefore almost impossible. To the extent possible, viable single cells were deposited from the FITC-positive gate (Figure 17).

Three weeks after single cell deposition, the first screening of the clones for the FITC signal followed and, thus, the presence of eGFP in the FACS. Previously, an average growth rate of 43% had been determined microscopically. A total of 952 clones were screened, of which 33 were identified as potentially positive clones. This corresponded to an efficiency of 3.4%. No clones could be identified in the three samples 1L, 3L and 3Z. Again, it was possible to see that the circular donor plasmid samples yielded more potentially positive clones than did samples with the linearized donor plasmid (Table 7).

**Table 7: Identification of potentially positive clones from the Knock-in Strategy V3 using FACS after single-cell deposition. The number of potentially positive clones identified after single cell deposition is stated for each gRNA and gate. The total number of examined clones is stated in parentheses. The efficiency resulted from the ratio of the number of potential clones to the number of clones examined.**

| | **Number of potential clones** | | **Efficiency** | |
|---|---|---|---|---|
| | Linear donor plasmid | Circular donor plasmid | Linear donor plasmid | Circular donor plasmid |
| gRNA1 | 0 (195) | 12 (62) | 0% | 19.4% |
| gRNA2 | 7 (195) | 14 (214) | 3.6% | 6.5% |
| gRNA3 | 0 (212) | 0 (105) | 0% | 0% |
| total | 7 (602) | 26 (381) | 1.2% | 6.8% |
| | **33 (983)** | | **3.4%** | |

The FITC histograms from the first FACS screen showed that the signal differences versus the wild-type were only very small. Some clones showed a relatively stronger shift in the FITC-positive region, but this was less than a log step.

In total, 33 clones with an elevated FITC signal could be identified and expanded for further analysis. During cultivation, the stability of the eGFP signal was regularly checked by FACS. After a few passages, it was found that most of the clones had completely lost the previously low FITC signal. In the overlay, their signal was identical to that of the wild-type.

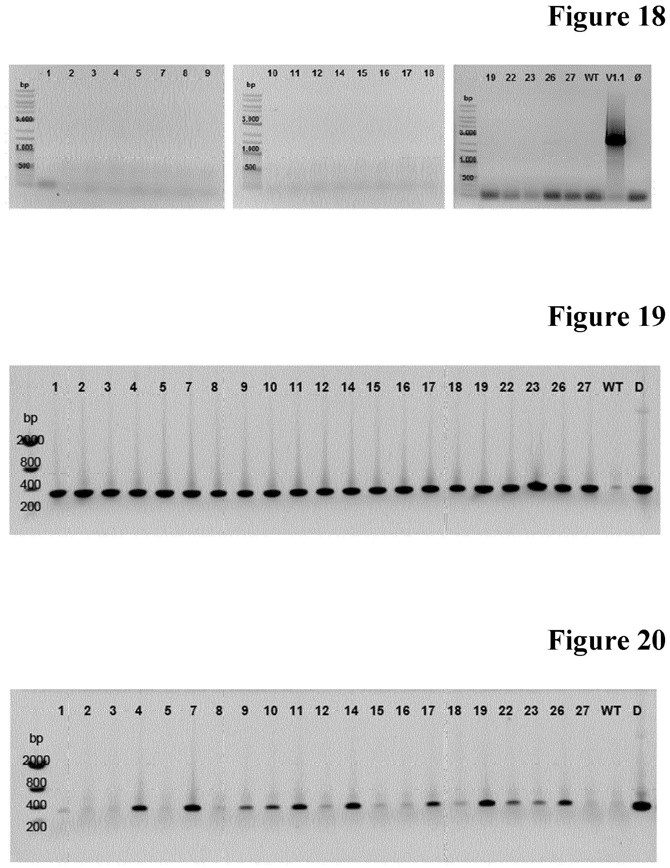

By PCR on the cDNA of the clones, the complete coding region of eGFP-TUBA1B could be amplified. For this purpose, the total RNA was isolated from the clones and transcribed into cDNA. The primers for the PCR were chosen so that a specific product is only formed when precise insertion of eGFP occurred. The PCR was analyzed in agarose gel. As expected, no product could be amplified in the wild-type sample. Likewise, no amplicon was detected in any of the clones. Clone 1 from the Knock-in Strategy V1 was carried along as a positive control for the PCR, which produced the specific product of 2085 bp. At the DNA level, no specific knock-in of eGFP could be shown for any of the analyzed clones (Figure 18).

Some clones were examined microscopically. The fixed and permeabilized cells were stained with the Alexa Fluor 647 conjugated anti-GFP antibody and Hoechst 33342. No detection of eGFP was possible in any of the clones. Likewise, no GFP signal, and accordingly no filamentary tubulin structures with eGFP-TUBA1B fusion protein, could be detected by antibody staining.

The reason why the clones survived despite selection with hygromycin B was determined by PCR on the cDNA of the clones with specific primers for eGFP or the hygromycin B-resistance cassette (SEQ ID NO: 26 and 27). As expected, no product could be amplified with the hygromycin B-specific primers in the wild-type test. The donor plasmid contained the hygromycin B-resistance cassette and therefore functioned as a positive control. A specific band could be amplified in PCR with primers for the hygromycin B-resistance cassette for all clones (Figure 19). The PCR with eGFP specific primers on the cDNA of the clones also showed no product in the wild-type. The donor plasmid contains the eGFP sequence without the start codon ATG, and served as a positive control with the chosen primers. In some clones, for example clones 4, 7, 14 and 19, a pronounced band appeared in the agarose gel at expected height. Other clones, such as clones 1 or 12, only showed one band of weak intensity. For clones 2, 3, 5, 8 and 27, no specific product could be detected in the agarose gel with this PCR (Figure 20).

### Summary of Knock-in Strategy V3:

In the Knock-in Strategy V3, the cells were transfected with two plasmids. The donor plasmid contained the eGFP sequence to be introduced surrounded by the homologous sequences around the insertion site. The second plasmid pCas-Guide-EF1α-CD4, referred to as an all-in-one plasmid, also encodes for a gRNA in addition to the Cas9 nuclease. In addition, a CD4 marker was present on the plasmid. The selection was based on CD4 pool sorting for Cas9 and gRNA, followed by hygromycin B selection for the donor plasmid. In total, out of 952 screened clones, 33 potentially positive clones were identified. The following experiments showed that all clones were negative clones. No precise knock-in at the desired locus could be detected at the DNA level by means of PCR.

The properties of the 33 clones analyzed are summarized in Table 8.

**Table 8: List of all the clones from the Knock-in Strategy V3 with the summarized results.**

| **No .** | **Clone** | **% FITC-positive first screen** | **% FITC-positive last screen** | **Western blot eGFP-TUBA1 B** | **Imaging** | **PCR hygro mycin** | **PCR eGFP** | **Sequ. cDNA** | **Pos. Clone** |
|---|---|---|---|---|---|---|---|---|---|
| 1 | 2L P2 F10 | 6.1 | 0.8 | Negative | Negative | Pos. | Pos. | Neg. | No |
| 2 | 2L P2 F12 | 13.4 | 0.8 | Negative | N/A | Pos. | Neg. | Neg. | No |
| 3 | 2L P3 E12 | 8.9 | 0.4 | Negative | N/A | Pos. | Neg. | Neg. | No |
| 4 | 2L P3 H10 | 11.9 | 0.2 | Negative | N/A | Pos. | Pos. | Neg. | No |
| 5 | 2L P4 C1 | 7.8 | 0.8 | Negative | N/A | Pos. | Pos. | Neg. | No |
| 7 | 2L P4 F1 | 14.5 | 0.4 | Negative | N/A | Pos. | Pos. | Neg. | No |
| 8 | 2L P4 H4 | 7.2 | 0.3 | Negative | Negative | Pos. | Pos. | Neg. | No |
| 9 | 2Z P1 A5 | 9.7 | 0.3 | Negative | N/A | Pos. | Pos. | Neg. | No |
| 10 | 2Z P2 A6 | 6.6 | 0.1 | Negative | N/A | Pos. | Pos. | Neg. | No |
| 11 | 2Z P2 A7 | 17 | 0.1 | Negative | N/A | Pos. | Pos. | Neg. | No |
| 12 | 2Z P2 A9 | 21.7 | 0.1 | Negative | N/A | Pos. | Pos. | Neg. | No |
| 14 | 2Z P3 A6 | 8.6 | 0.1 | Negative | N/A | Pos. | Pos. | Neg. | No |
| 15 | 2Z P3 A7 | 8.9 | 0.1 | Negative | N/A | Pos. | Pos. | Neg. | No |
| 16 | 2Z P4 A4 | 30.9 | 0.1 | Negative | N/A | Pos. | Pos. | Neg. | No |
| 17 | 2Z P4 A11 | 7.7 | 0.1 | Negative | N/A | Pos. | Pos. | Neg. | No |
| 18 | 2Z P4 E12 | 30.2 | 0.1 | Negative | N/A | Pos. | Pos. | Neg. | No |
| 19 | 2Z P4 H4 | 7.8 | 2.6 | Negative | Negative | Pos. | Pos. | Neg. | No |
| 22 | 2Z P1 A7 | 8.6 | 0.2 | Negative | N/A | Pos. | Pos. | Neg. | No |
| 23 | 2ZP1 A10 | 19.1 | 0.2 | Negative | N/A | Pos. | Pos. | Neg. | No |
| 26 | 2Z P2 A2 | 10.2 | 0.2 | Negative | N/A | Pos. | Pos. | Neg. | No |
| 27 | 2Z P2 A4 | 31 | 0.1 | Negative | N/A | Pos. | Neg. | Neg. | No |
| 28 | 1Z P1 A3 | 13.7 | 0.3 | Negative | N/A | N/A | N/A | N/A | No |
| 29 | 1Z P1 A4 | 26.3 | 0.3 | Negative | N/A | N/A | N/A | N/A | No |
| 30 | 1Z P1 A6 | 9 | 0.2 | Negative | N/A | N/A | N/A | N/A | No |
| 31 | 1ZP1 A11 | 16.9 | 0.1 | Negative | N/A | N/A | N/A | N/A | No |
| 32 | 1Z P1 B8 | 26.4 | 2.1 | Negative | N/A | N/A | N/A | N/A | No |
| 33 | 1ZP1 B10 | 16 | 1.5 | Negative | N/A | N/A | N/A | N/A | No |
| 34 | 1Z P1 C4 | 10.8 | 1.5 | Negative | N/A | N/A | N/A | N/A | No |
| 35 | 1Z P1 C7 | 21.2 | 0.4 | Negative | N/A | N/A | N/A | N/A | No |
| 36 | 1Z P1 D1 | 20.6 | 0.1 | Negative | N/A | N/A | N/A | N/A | No |
| 37 | 1Z P1 D3 | 13.2 | 0.5 | Negative | N/A | N/A | N/A | N/A | No |
| 38 | 1Z P1 D9 | 13.5 | 0.3 | Negative | N/A | N/A | N/A | N/A | No |
| 39 | 1ZP1 D10 | 13.4 | 0.5 | Negative | N/A | N/A | N/A | N/A | No |

### 4) Discussion

The generation of a specific knock-in with CRISPR/Cas9 for the precise modification of endogenous proteins proved to be non-trivial. By way of example, this was demonstrated by means of an N-terminal knock-in of eGFP to the TUBA1B model gene in HEK293A cells.

Such a GFP knock-in on TUBA1B has already been realized by several groups, for example in U2O2 osteosarcoma cells using zinc finger nucleases (16) or in human inducible pluripotent stem cells (iPSC) with CRISPR/Cas9 (17). Roberts et al. analyzed the structure of the N-terminally tagged TUBA1B in human iPSCs directly via the GFP tag in live cell time-lapse imaging.

The herein exemplified knock-in was based on the same basic strategy, which was carried out in three different variants: V1, V2 and V3.

In Knock-in Strategy V2, the FACS screening of the clones showed distinctly more heterogeneous intensities in the eGFP signal. In particular, the clones with the crRNA3 and the circular donor plasmid showed a remarkably strong eGFP signal stability after long-term cultivation with over 87% of the cells in the FITC-positive range. The clones with crRNA1 and the circular donor plasmid showed a broad diversity by FACS, as between 21% and 75% of the cells were in the FITC-positive range.

With the Knock-in Strategy V3, it was not possible to generate positive clones at all. In the first FACS screening, clones were selected due to the small proportion of cells with detectable eGFP signal, in which more than 5% of the cells were in the FITC-positive range. In these clones, there was no discernible shift of the whole cell population into the FITC-positive region. The analyses during the continuous cultivation showed that after a few passages, a eGFP signal could not be detected anymore. At the DNA level, no knock-in of the eGFP sequence could be detected in front of the TUBA1B gene. It was likely that a false positive signal was measured on these cells in the first FACS screen. PCR with hygromycin B-specific primers produced positive results in all clones, indicating that the clones were able to survive antibiotic selection. In addition, part of the eGFP sequence was detected in many clones by PCR. These results suggested that the identified potentially positive clones randomly integrated the donor plasmid into their genome. The eGFP sequence did not carry its own promoter or start codon on the donor plasmid. Therefore, it is unlikely that the eGFP will randomly integrate into the correct reading frame in a transcribed locus. No precise knock-in could be detected in any of the clones.

In some of the clones from knock-in strategies V1 and V2, a monoallelic knock-in was detected by PCR on cDNA (data not shown) (see also 17).

Comparing the effectiveness of the three tested knock-in strategies V1, V2, and V3, it was found that the double selection with hygromycin B and puromycin showed the highest efficiency. A correct eGFP knock-in could be verified for 29 of the 40 potentially positive clones. Thus, 17.5% of the clones identified as potentially positive were false positives and 72.5% were actually positive. If this is applied to the originally identified 118 potentially positive clones, 86 positive clones are to be expected among the 496 analyzed clones. This corresponds to a total efficiency of 17.3%. In the literature, widely varying efficiencies for homologous recombination are found in the range of 0.1% to 5%. Efficiencies of up to 24% are also indicated under optimized conditions and depending on the respective locus (17, 18).

One of the most important factors for the efficiency of homologous recombination is the donor template. In the strategies tested, the plasmids used were circular or linearized through restriction with NotI. The results showed that a lower knock-in efficiency could be achieved with linearized donor plasmid than with the circular plasmid. The various donor variants were tested and compared in numerous articles. Stieger et al. showed that, more indel mutations could be identified in the target locus with a 3'-overhang linear donor than with a circular or linearized donor with a 5'-overhang. Similar findings have been described by Liang et al. They showed that in a linear double-stranded donor with 30 nucleotide overhangs, a significantly better HDR efficiency could be achieved than with a donor without an overhang (19). Zhang et al. used a circular as well as double-cut donor in HEK293T cells in which the plasmid backbone was completely removed. With the circular donor an HDR efficiency of 5% was achieved, with the linear double-cut donor even 21%. The double-cut donor significantly increases the knock-in rate and requires significantly shorter homologous sequences than does the circular donor plasmid (20). The working group of Chu et al. achieved the best knock-in results in HEK293 cells with a generated PCR product having 1 kb homologous sequences as a template (21).

In order to obtain cells with a successful knock-in, precise selection of non-edited wild-type cells is required. In the variants used, antibiotic selection was most efficient when done on both plasmids.

PCR analysis showed that in strategy V1, only two of 23 clones showed no Cas9 sequence.

Benefits were expected from the all-in-one pCas-Guide-EF1α-CD4, as CD4 selection served only to isolate the transfected cells. CD4 sorting did not artificially force the cells to retain the plasmid in order to survive, and they lost it after about 7 days.

In Roberts et al., the selection was made exclusively via the GFP signal by FACS and despite the absence of antibiotics, fortuitous integration of the donor plasmid was detected in 45% of the clones, depending on the crRNA that was used (17).

The advantage of the eGFP tag is that it provides signals even without additional staining with an antibody, which, for example, is more economical in terms of time and cost in the FACS screening of the clones. Alternatively, the smaller Myc Tag can be used.

### Figures

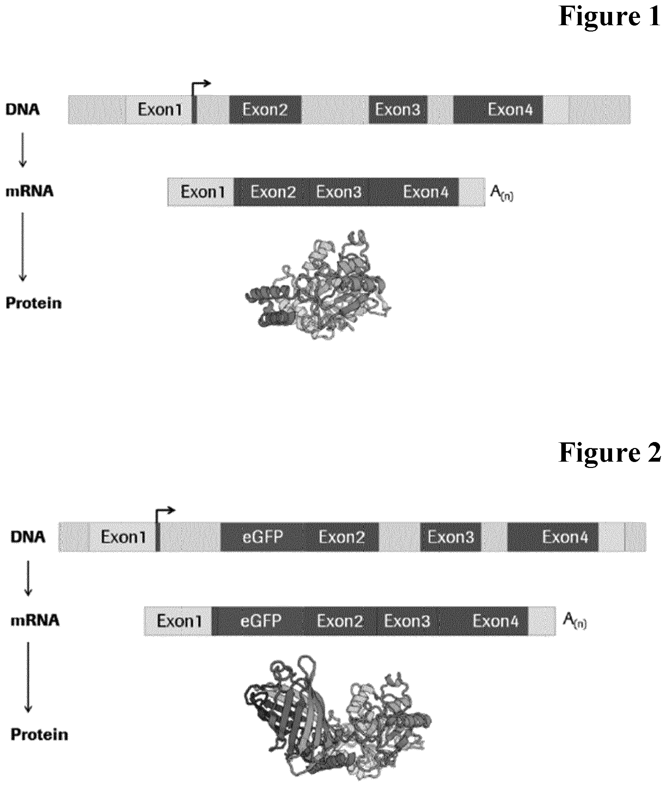

- **Figure 1**: Schematic representation of the human TUBA1B locus in the genome prior to the insertion of an N-terminal eGFP tag with the respective transcribed mRNA and the protein translated from it. The coding sequence (dark blue) is flanked by untranslated regions (light blue), as well as intron sequences (gray).
- **Figure 2**: Schematic representation of the human TUBA1B locus in the genome after insertion of an N-terminal eGFP tag with the transcribed mRNA and the protein translated from it. The coding sequence (dark blue) is flanked by untranslated regions (light blue), as well as intron sequences (gray).

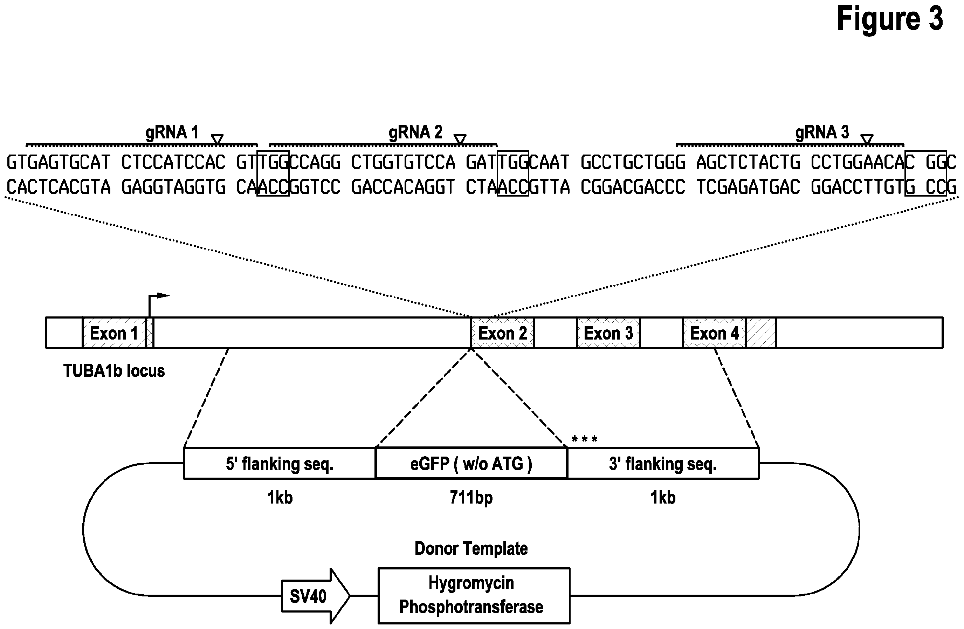

- **Figure 3**: Knock-in strategy for introducing the eGFP sequence upstream of exon 2 at the genomic TUBA1B locus. The three specific gRNAs bind at the beginning of exon 2 and generate the double strand break with Cas9. The donor plasmid contains the eGFP sequence to be inserted, surrounded by 1 kb homologous sequences for homologous recombination. The asterisks (*) on the 3'-homologous sequence of the donor plasmid identify point mutated regions of the gRNA binding sites.

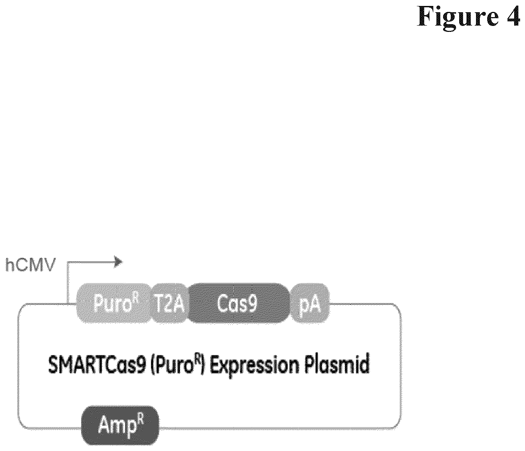

- **Figure 4**: Plasmid map for Cas9-PuroR plasmid (Dharmacon).

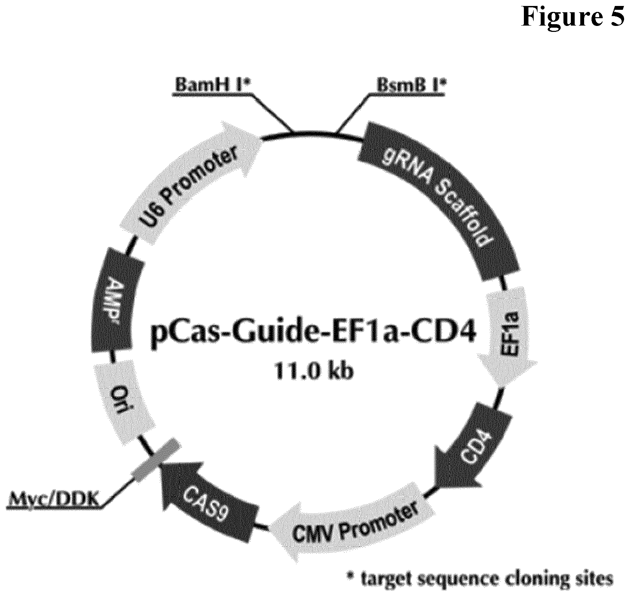

- **Figure 5**: Plasmid map for pCas9-Guide-EF1α-CD4 plasmid (Origene)

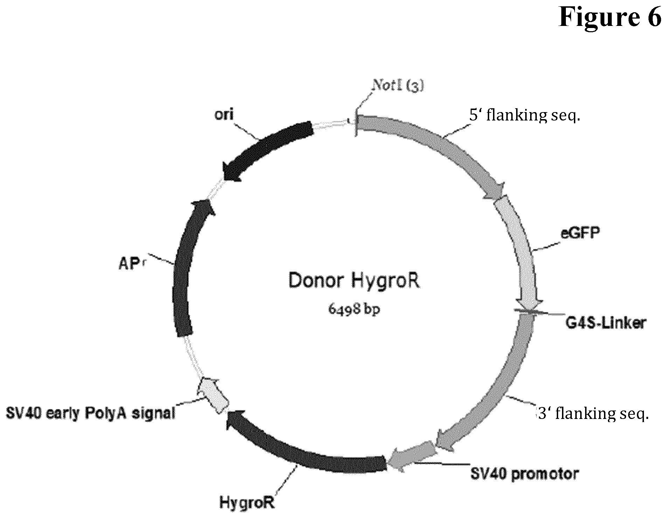

- **Figure 6**: Plasmid map for the donor plasmid with hygromycin B-resistance cassette

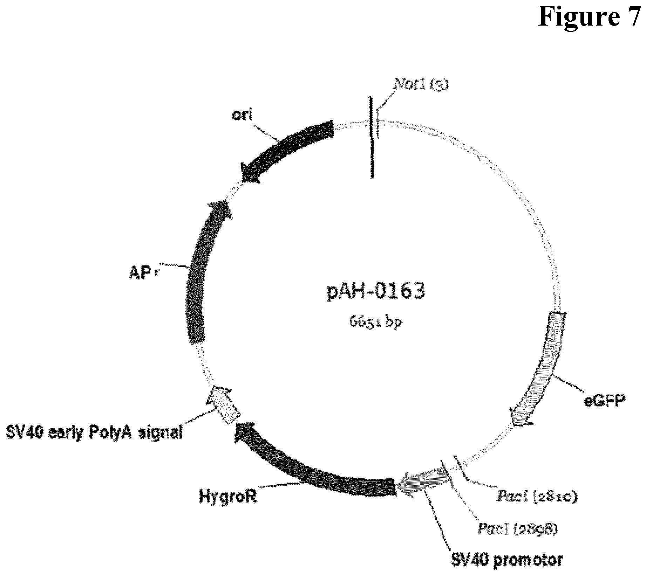

- **Figure 7**: Plasmid map eGFP hygromycin B plasmid used to prepare the donor plasmids.
- **Figure 8**: HEK293A wild-type cells were transfected with two TUBA1B-specific crRNAs, tracrRNA, the circular donor plasmid and the Cas9-PuroR plasmid. After selection with puromycin, single cells were deposited in a 96-well format. Shown here is the gating strategy for the crRNA1 sample. Gating was done on viable single cells via the FSC and SSC channels. It was possible to detect the presence of eGFP via the FITC channel. The FITC signal of the samples (blue) is normalized in histogram together with the wild-type (black) and shown zoomed in. Single cells were deposited from the labeled gates P3 and P4.
- **Figure 9**: HEK293A wild-type cells were transfected with two TUBA1B-specific crRNAs, synthetic tracrRNA, the circular donor plasmid and the Cas9-PuroR plasmid. After selection with puromycin, single cells were deposited in a 96-well format. The gating strategy for the crRNA2 sample is shown. Gating was done on viable single cells via the FSC and SSC channels. It was possible to detect the presence of eGFP via the FITC channel. The FITC signal of the samples (blue) is normalized in histogram together with the wild-type (black) and shown zoomed in. Single cells were deposited from the labeled gates P3 and P4.
- **Figure 10**: PCR on the cDNA of the clones from Knock-in Strategy V1 to check the knock-in at the genomic level. Schematic representation of the cDNA of TUBA1B and eGFP-TUBA1B, as well as a part of the donor plasmid. The arrows indicate the binding sites of the primers that are used. Both primers can only bind when a precise knock-in of the eGFP in front of TUBA1B occurred. The coding sequence is marked in light blue and the untranslated regions in gray.
- **Figure 11**: PCR on the cDNA of the clones from Knock-in Strategy V1 to check the knock-in at the genomic level. The PCR on the cDNA of the clones (1 to 23) and the wild-type (WT) with the primers eGFP fwd and TUBA1B Exon4 rev was applied to an agarose gel. In the case of a precise knock-in, a product of 999 bp should be amplified.
- **Figure 12**: Analysis of the functionality of the eGFP-TUBA1B protein in the clones from Knock-in Strategy V1 by means of imaging. The cells were fixed, permeabilized and stained with a GFP-Alexa Fluor 647 antibody. The nuclei were stained with Hoechst 33342. The endogenous eGFP was stimulated at 488 nm, the Alexa Fluor 647 from the GFP antibody at 647 nm and the bound Hoechst 33342 dye at 355 nm. From each clone, 40 pixels were recorded with the 40× objective lens. Representative image sections are shown in each case.
- **Figure 13**: Single-cell deposition of transfected samples from Knock-in Strategy V2. HEK293A wild-type cells were transfected with three TUBA1B-specific crRNAs, tracrRNA, the circular or linearized donor plasmid and the Cas9-PuroR plasmid. After double selection with hygromycin B and puromycin, single cells were deposited in 96-well format. The FITC histograms of the crRNA1 sample 1L, the crRNA2 sample 2L and the crRNA3 sample 3L are shown, each with linearized donor plasmid, as well as the crRNA1 sample 1Z, the crRNA2 sample 2Z and the crRNA3 sample 3Z each with circular donor plasmid. Gating was done on viable single cells via the FSC and SSC channels. It was possible to detect the presence of eGFP via the FITC channel. The FITC signal of the samples (blue) is normalized in the histogram in the overlay with the wild-type (black) and enlarged. Single cells from the FITC-positive gate were deposited.
- **Figure 14**: Analysis of the complete eGFP-TUBA1B sequence of the clones from the knock-in V2. The PCR on the cDNA of the clones and the wild-type (WT) with the primers cDNA fwd and cDNA rev was applied to an agarose gel. This PCR was designed to amplify the entire coding region of eGFP-TUBA1B and result in a product of 2085 bp at knock-in.
- **Figure 15**: Analysis of the functionality of the eGFP-TUBA1B in the clones of the Knock-in Strategy V2 by means of imaging. The cells were fixed, permeabilized and stained with a GFP-Alexa Fluor 647 antibody. The nuclei were stained with Hoechst 33342. The endogenous eGFP was stimulated at 488 nm, the staining with the GFP-Alexa Fluor 647 antibody at 647 nm, and the bound Hoechst 33342 dye at 355 nm. From each clone, 40 pixels were recorded with the 20× objective. Representative image sections are shown here.
- **Figure 16**: Pool sorting of CD4 positive cells of the transfected samples from the Knock-in Strategy V3. HEK293A wild-type cells were transfected with one of three pCas-Guide EF1α-CD4 plasmids with different gRNA sequences and the circular or linearized donor plasmid. After 48 h, a pool of CD4 positive cells was deposited. The transfected samples from gRNA1/2/3 contained the pCas-Guide-EF1α-CD4 plasmid with the linear donor (1L/2L/3L) and those from gRNA1/2/3 contained the pCas-Guide-EF1α-CD4 with the circular donor plasmid (1Z/2Z/3Z), and were stained with an APC-conjugated anti-CD4 antibody. The histograms showed the normalized APC signal of the stained sample (blue) in an overlay with the stained wild-type (black). Cells from the APC positive gate were sorted as a pool from the cell suspension.
- **Figure 17**: Single cell deposition of the CD4-positive pool samples from the Knock-in Strategy V3. The samples were selected 24 h after CD4 pool sorting with hygromycin B. Single cells from the cells grown were deposited in a 96-well format. The FITC histograms of the gRNA1 sample 1L, the gRNA2 sample 2L and the gRNA3 sample 3L are shown, each with the linearized donor plasmid, and the gRNA1 sample 1Z, the gRNA2 sample 2Z and the gRNA3 sample 3Z each with the circular donor plasmid. Gating was done on viable single cells via the FSC and SSC channels. It was possible to detect the presence of eGFP via the FITC channel. The FITC signal of the samples (blue) is normalized in the histogram in the overlay with the wild-type (black) and zoomed in. Single cells were deposited from the FITC-positive gate.
- **Figure 18**: Analysis of the complete eGFP-TUBA1B sequence of the clones from the Knock-in Strategy V3. The PCR on the cDNA of the clones and the wild-type (WT) with the primers cDNA fwd and cDNA rev was applied to an agarose gel. With this PCR, the entire coding region of eGFP-TUBA1B should be amplified to give a product of 2085 bp when a precise knock-in occurred.
- **Figure 19**: PCR of the clones of Knock-in Strategy V3. PCR on the cDNA of the clones (1 to 27), the wild-type (WT) and the donor plasmid (D) with the primers Hygro fwd (SEQ ID NO: 26) and Hygro rev (SEQ ID NO: 27) for amplifying a region from the hygromycin B-resistance cassette. The specific product has a size of 324 bp.
- **Figure 20**: PCR of the clones of Knock-in Strategy V3. PCR on the cDNA of the clones (1 to 27), the wild-type (WT) and the donor plasmid (D) with the primers eGFP_2 fwd and eGFP_2 rev for the amplification of an area from the eGFP sequence. The specific product has a size of 300 bp.

### Sequences

| | |
|---|---|
| gRNA1 forward | SEQ ID NO: 01 |
| gRNA1 reverse | SEQ ID NO: 02 |
| gRNA2 forward | SEQ ID NO: 03 |
| gRNA2 reverse | SEQ ID NO: 04 |
| gRNA3 forward | SEQ ID NO: 05 |
| gRNA3 reverse | SEQ ID NO: 06 |
| crRNA1 | SEQ ID NO: 07 |
| crRNA2 | SEQ ID NO: 08 |
| crRNA3 | SEQ ID NO: 09 |
| D_Hygro fwd | SEQ ID NO: 10 |
| D_Hygro rev | SEQ ID NO: 11 |
| D_5' HA fwd | SEQ ID NO: 12 |
| D_5' HA rev | SEQ ID NO: 13 |
| D_eGFP fwd | SEQ ID NO: 14 |
| D_eGFP_rev | SEQ ID NO: 15 |
| D_3' HA fwd | SEQ ID NO: 16 |
| D_3' HA rev | SEQ ID NO: 17 |
| D_3' HA-Z fwd | SEQ ID NO: 18 |
| D_3' HA-Z fwd | SEQ ID NO: 19 |
| QuickChange fwd | SEQ ID NO: 20 |
| QuickChange rev | SEQ ID NO: 21 |
| eGFP fwd | SEQ ID NO: 22 |
| TUBA1B exon4rev | SEQ ID NO: 23 |
| cDNA fwd | SEQ ID NO: 24 |
| cDNA rev | SEQ ID NO: 25 |
| Hygro fwd | SEQ ID NO: 26 |
| Hygro rev | SEQ ID NO: 27 |
| Cas9 fwd | SEQ ID NO: 28 |
| Cas9 rev | SEQ ID NO: 29 |
| eGFP_2 fwd | SEQ ID NO: 30 |
| eGFP_2 rev | SEQ ID NO: 31 |
| eGFP tag amino acid sequence | SEQ ID NO: 32 |
| eGFP tag nucleotide sequence | SEQ ID NO: 33 |

### Literature

(1) Sternberg S, Doudna J. Expanding the Biologist's Toolkit with CRISPR-Cas9. Molecular Cell 58, 568-574 (2015).
(2) Rice A, Hornblower B, Robb B, Tzwetzinis G. CRISPR/Cas9 and Targeted Genome Editing: A New Era in Molecular Biology. NEB expressions Issue I. 2014; Available at: https://www.neb.com/tools-and-resources/feature-articles/crispr-cas9-and-targeted-genome-editing-an-new-era-in-molecular-biology. Accessed May 26, 2017.
(3) Jo Y, Suresh B, Kim H, Ramakrishna S. CRISPR/Cas9 system as an innovative genetic engineering tool: Enhancements in sequence specificity and delivery methods. Biochimica et Biophysica Acta (BBA) - Reviews on Cancer. 2015; 1856 (2): 234-243.
(4) Jinek M, Chylinski K, Fonfara I, Hauer M, Doudna J, Charpentier E. A Programmable Dual RNA-Guided DNA Endonuclease in Adaptive Bacterial Immunity. Science. 2012; 337 (6096): 816-821.
(5) Cong L, Ran F, Cox D, Lin S, Barretto R, Habib N et al. Multiplex Genome Engineering Using CRISPR/Cas Systems. Science. 2013; 339 (6121): 819-823.
(6) Chavez A, Doench J, Esvelt K, Hough S, Karve A. CRISPR 101: A desktop resource. Available at: http://info.addgene.org/download-addgenes-ebook-crispr-101-2nd-edition. Accessed May 19, 2017.
(7) Graw J. Genetics. 1st ed. Berlin: Springer; 2010.
(8) Ran F, Hsu P, Wright J, Agarwala V, Scott D, Zhang F. Genome engineering using the CRISPR-Cas9 system. Nature Protocols. 2013; 8 (11): 2281-2308.
(9) Hsu P, Scott D, Weinstein J, Ran F, Konermann S, Agarwala V et al. DNA targeting specificity of RNA-guided Cas9 nucleases. Nature Biotechnology. 2013;31 (9):827-832.
(10) Mali P, Yang L, Esvelt K, Aach J, Guell M, DiCarlo J et al. RNA-Guided Human Genome Engineering via Cas9. Science. 2013; 339 (6121): 823-826.
(11) Mojica F, Díez-Villaseñor C, Garcia-Martinez J, Almendros C. Short motif sequences determine the targets of the prokaryotic CRISPR defense system. Microbiology. 2009; 155 (3): 733-740.
(12) Hsu P, Lander E, Zhang F. Development and Applications of CRISPR-Cas9 for Genome Engineering. Cell. 2014; 157 (6): 1262-1278.
(13) Lee J, Grav L, Lewis N, Faustrup Kildegaard H. CRISPR/Cas9-mediated genome engineering of CHO cell factories: Application and perspectives. Biotechnology Journal. 2015; 10 (7): 979-994.
(14) Pellagatti A, Dolatshad H, Valletta S, Boultwood J. Application of CRISPR/Cas9 genome editing to the study and treatment of disease. Archives of Toxicology. 2015; 89 (7): 1023-1034.
(15) Janke C. The tubulin code: Molecular components, readout mechanisms, and functions. J Cell Biol. 2014; 206 (4): 461-472. doi: 10.1083/jcb.201406055.
(16) Pruett-Miller S. Chromosomal mutagenesis. New York: Humana Press; 2015.
(17) Roberts B, Main A, Tucker A et al. Systematic gene tagging using CRISPR/Cas9 in human stem cell to illuminate cell organization. Mol Biol Cell. 2017; 28 (21): 2854-2874. doi: 10.1091/mbc.e17-03-0209.
(18) Maruyama T, Dougan S, Truttmann M, Bilate A, Ingram J, Ploegh H. Increasing the efficiency of precise genome editing with CRISPR-Cas9 by inhibition of nonhomologous end-joining. Nat Biotechnol. 2015; 33 (5): 538-542. doi: 10.1038/nbt.3190.
(19) Liang X, Potter J, Kumar S, Ravinder N, Chesnut J. Enhanced CRISPR/Cas9-mediated precise genome editing by improved design and delivery of gRNA, Cas9 nuclease, and donor DNA. J Biotechnol. 2017; 241: 136-146. doi: 10.1016/j.jbiotec.2016.11.011.
(20) Sander J, Joung J. CRISPR-Cas systems for editing, regulating and targeting genomes. Nat Biotechnol. 2014; 32 (4): 347-355. doi: 10.1038/nbt.2842.
(21) Chu V, Weber T, Wefers B et al. Increasing the efficiency of homology-directed repair for CRISPR-Cas9-induced precise gene editing in mammalian cells. Nat Biotechnol. 2015; 33 (5): 543-548. doi: 10.1038/nbt.3198.

### Material and methods

### Consumables:

| **Material** | **Manufacturer** | **Use** |
|---|---|---|
| 96-well cell culture plate, black with transparent bottom | Corning Life Science | CellTiter-Glo assay |
| 96-well cell culture plate, black with transparent bottom, collagen coated | Corning Life Science | Imaging in the Operetta system |
| Blot paper | BioRad | Western blot |
| CryoPure Tube (1.8 mL) | Sarstedt | cell culture |
| Eppendorf tube (0.5 mL/1.5 mL/2 mL) | Eppendorf | general |
| Falcon conical centrifuge tubes, 15 mL/50 mL | Corning Life Science | general |
| Falcon Round Bottom Tubes with Cell Sieve Cap, 5 mL | Corning Life Science | FACS |
| Falcon Round Bottom Tubes, 14 mL | Corning Life Science | Mini prep |
| Falcon round bottom tube, 5 mL | Corning Life Science | FACS |
| Slide 96-well plate | Eppendorf | general |
| Microplate 96-well, U-bottom | Corning Life Science | FACS |
| Microplate 96-well, V-bottom | Corning Life Science | FACS |
| Multi-well cell culture plates, clear, flat bottom, TC (6-well, 24-well, 96-well) | Corning Life Science | Cell culture, transfection |
| Nitrocellulose membrane (0.2 µm) | Invitrogen | Western blot |
| PCR reaction vessel (0.2 mL) | Eppendorf | PCR |
| PCR reagent vessel chain (0.2 mL) | Eppendorf | PCR |
| Petri dishes | Sarstedt | cloning |
| Pipette tips (10 µL, 200 µL, 1 mL) | Eppendorf | general |
| Plating applicator | Sarstedt | cloning |
| Serological pipettes (5 mL, 10 mL, 25 mL, 50 mL) | Sarstedt | general |
| Cell culture bottle (T25/T75) | Sarstedt | cell culture |

### Chemicals:

| **Chemical** | **Manufacturer** | **Use** |
|---|---|---|
| 100 bp DNA marker | Biomol | cloning |
| 1 kb DNA marker | Biomol | cloning |
| Agarose I | ThermoFisher | agarose gels |
| ampicillin | Sigma | cloning |
| Complete ULTRA Tablet Mini | Sigma | Western blot |
| CutSmart buffer (10×) | NEB | cloning |
| DMSO | Sigma | cell culture |
| ethanol | Sigma | general |
| Ethidium bromide solution (10 mg/mL) | Sigma-Aldrich | agarose gels |
| Gel purple loading dye (6×) | NEB | cloning |
| Hoechst 33342 (10 mg/mL) | Molecular probes | Imaging in the Operetta system |
| isopropanol | Sigma | general |
| Kanamycin sulfate | Gibco | cloning |
| LB agar, powder | Invitrogen | cloning |
| LB Broth | Gibco | cloning |
| methanol | Sigma | Western blot |
| Milk powder, low-fat | Carl Roth | Western blot |
| NEB 3.1 buffer (10×) | NEB | cloning |
| NuPAGE Antioxidant | Invitrogen | Western blot |
| NuPAGE LDS sample buffer (4×) | Invitrogen | Western blot |
| NuPAGE MOPS SDS running buffer (20×) | Invitrogen | Western blot |
| NuPAGE transfer buffer (20×) | Invitrogen | Western blot |
| Reducing agent (10×) | Invitrogen | Western blot |
| RIPA buffer (10×) | Merck | Western blot |
| Hydrochloric acid 37% | Merck | general |
| trypan blue | Invitrogen | cell culture |
| Tween 20 | Sigma | Western blot |
| UltraPure DNA typing TAE buffer (50×) | Invitrogen | agarose gels |

### Plasmids:

Cas9 plasmids were purchased from Dharmacon (Figure 4) and Origene (Figure 5). The donor plasmids were self-cloned (Figure 6 (with hygromycin B-resistance cassette), Figure 7 (pAH-0163)). All plasmids were stored at -20 °C.

### Enzymes:

All enzymes used were purchased from NEB and stored at -20 °C.

| **Enzyme** | **Temperature for maximum enzyme activity (°C)** | **Use** |
|---|---|---|
| BamHI-HF | 37 | Cloning donor plasmids |
| BsmBI | 55 | Cloning pCas-Guide-EF1a-CD4 plasmid |
| DpnI | 37 | Site-directed mutagenesis |
| HindIII-HF | 37 | Cloning donor plasmids |
| NaeI | 37 | Cloning donor plasmids |
| NotI HF | 37 | Linearization donor plasmid with hygromycin B-resistance cassette |
| PacI | 37 | Cloning donor plasmids |
| PvuI HF | 37 | Cloning donor plasmids |
| rSAP | 37 | Dephosphorylation of the vector ends |
| Spel HF | 37 | Cloning donor plasmids |
| T4 DNA ligase | 16 | ligation |
| XmaI | 37 | Cloning donor plasmids |

### Buffers and solutions:

| **Designation** | **Composition** | **pH value** | **Use** |
|---|---|---|---|
| 1 × Tris-acetate-EDTA buffer (TAE) | 20 mL 50× TAE buffer make up with ddH₂O to 1 liter | - | Preparation of agarose gel |
| FACS buffer | 50 mL FCS make up with 1 × PBS to 500 mL | - | Analytical FACS, Cell Sorting |
| 1 × RIPA buffer | 1 mL 10× RIPA buffer | - | Production of protein lysates |
| | 9 mL ddH₂O | | |
| | 1 complete ULTRA Tablets, mini | | |
| 2.8 × LDS sample buffer | 280 µL 4× LDS sample buffer | 8.4 | Western blot |
| | 110 µL 10× reducing agent | | |
| 1 × running buffer | 25 mL 20× NuPAGE MOPS SDS running buffer | 7.7 | Western blot |
| | 500 µL NuPAGE antioxidant make up with ddH₂O to 500 mL | | |
| 1× transfer buffer | 25 mL 20× NuPAGE transfer buffer | 8.3 | Western blot |
| | 100 mL of methanol make up with ddH₂O to 500 mL | | |
| 10× Tris-buffered saline (TBS) | 175.1 g of NaCl | 7.5 | Western blot |
| | 157.6 g Tris HCl | | |
| | × mL 37% HCl (to pH 7.6) make up with ddH₂O to 2 L | | |
| 1× Tris-buffered saline with Tween20 (TBST) | 200 mL 10× TBS | 7.5 | Western blot |
| | 1 mL Tween20 make up with ddH₂O to 2 L | | |
| 5% block solution | 5g skim milk powder | - | Western blot |
| | 100 mL 1×TBST | | |
| GSDB dyeing buffer | 16.7% Goat serum | - | Imaging in the Operetta system |
| | 0.3% Triton-X-100 | | |
| | 20 mM Na₃PO₄ | | |
| | 450 mM NaCl | | |
| High-salt sodium phosphate buffer | 130 mM NaCl | 7.4 | Imaging in the Operetta system |
| | 10 mM Na₃PO₄ | | |
| Low-salt sodium phosphate buffer | 300 mM NaCl | 7.4 | Imaging in the Operetta system |
| | 20 mM Na₃PO₄ | | |

### Oligonucleotides:

### TUBA1B specific crRNAs and gRNAs

| **Designation** | **5'-3' sequence** | **Manufactu rer** | **Usage** |
|---|---|---|---|
| gRNA1 forward SEQ ID NO: 01 | gatcgGAGTGCATC TCCATCCACGTg | metabion | ssDNA for Origen pCas9-Guide-EF1a-CD4 |
| gRNA1 reverse SEQ ID NO: 02 | aaaacACGTGGATG GAGATGCACTCc | metabion | ssDNA for Origen pCas9-Guide-EF1a-CD4 |
| gRNA2 forward SEQ ID NO: 03 | gatcgGGCCAGGCT GGTGTCCAGATg | metabion | ssDNA for Origen pCas9-Guide-EF1a-CD4 |
| gRNA2 reverse SEQ ID NO: 04 | aaaacATCTGGACA CCAGCCTGGCCc | metabion | ssDNA for Origen pCas9-Guide-EF1a-CD4 |
| gRNA3 forward SEQ ID NO: 05 | gatcgGAGCTCTAC TGCCTGGAACAg | metabion | ssDNA for Origen pCas9-Guide-EF1a-CD4 |
| gRNA3 reverse SEQ ID NO: 06 | aaaacTGTTCCAGG CAGTAGAGCTCc | metabion | ssDNA for Origen pCas9-Guide-EF1a-CD4 |
| crRNA1 SEQ ID NO: 07 | GAGTGCATCTCC ATCCACGT | Dharmacon | synthetic crRNA |
| crRNA2 SEQ ID NO: 08 | GGCCAGGCTGGT GTCCAGAT | Dharmacon | synthetic crRNA |
| crRNA3 SEQ ID NO: 09 | GAGCTCTACTGC CTGGAACA | Dharmacon | synthetic crRNA |

### Primer:

All primers were purchased from metabion in dissolved form at a concentration of 100 µM and stored at -20 °C.

| **Designation** | **5'-3' sequence** | **Usage** |
|---|---|---|
| D_Hygro fwd SEQ ID NO: 10 | CGAATGCGGCCGCAGAAGC AATAAGAGGACTGCGGAA GAGCTCCCTGTCAATGTA | Cloning donor with hygro resistance |
| D_Hygro rev SEQ ID NO: 11 | GCCTTAATTAAAGTGCTCC AGGGTGGTGTGGGTGGTGA GGATGGAGTTGT | Cloning donor with hygro resistance |
| D_5' HA fwd SEQ ID NO: 12 | GACATTGATTATTGAAAGC AATAAGAGGACTGCGGAA GAG | Cloning donor 5'HA |
| D_5' HA rev SEQ ID NO: 13 | TGCTCACCTGCGGGAAGGA AAAAAGATATCACAATTTA AA | Cloning donor 5'HA |
| D_eGFP fwd SEQ ID NO: 14 | TCCCGCAGGTGAGCAAGGG CGAGGAACTGTTCACCGGG GT | Cloning donor eGFP |
| D eGFP rev SEQ ID NO: 15 | ACTCACGGCTGCCTCCCCC GCCTTTGTACAGTTCGTCCA TTCCGA | Cloning donor eGFP |
| D_3' HA fwd SEQ ID NO: 16 | AAAGGCGGGGGAGGCAGC CGTGAATGTATAAGTATAC ATGTGGGACAAGCAGGAGT ACAAATCGGCAATGCCTGC TGGGA | Cloning donor 3'HA |
| D_3' HA rev SEQ ID NO: 17 | CTTTTGCTCACGGCCAGTG CTCCAGGGTGGTGTGGGTG GT | Cloning donor 3'HA |
| D_3' HA-Z fwd SEQ ID NO: 18 | GTCATCAATAGATTGGTTT AAATTGTGATATCTTTTTTC CTTCCCGCAG | Cloning donor 3HA |
| D_3' HA-Z fwd SEQ ID NO: 19 | AACCAGAAAGCTTTAACGT CTGTCAGTTAAGCTGAAGC TGAAATTCTGGG | Cloning donor 3'HA |
| QuickChange fwd SEQ ID NO: 20 | TGCCTGCTGGGAATTATAT TGTTTAGAGCATGGCATCC AG | Site-directed mutagenesis of the gRNA3 locus in the donor |
| QuickChange rev SEQ ID NO: 21 | CTGGATGCCATGCTCTAAA CAATATAATTCCCAGCAGG CA | Site-directed mutagenesis of the gRNA3 locus in the donor |
| eGFP fwd SEQ ID NO: 22 | GGCCGACAAGCAGAAAAA CGGCATCAAAGTGAAC | Characterization of the clones |
| TUBA1B Exon4 rev SEQ ID NO: 23 | GGCGGTTAAGGTTAGTGTA GGTTGGG | Characterization of the clones |
| cDNA fwd SEQ ID NO: 24 | ATGGTGAGCAAGGGCGAG GAACTGTTCACCGGGG | Characterization of the clones |
| cDNA rev SEQ ID NO: 25 | TTAGTATTCCTCTCCTTCTT CCTCACCC | Characterization of the clones |
| Hygro fwd SEQ ID NO: 26 | ACCGCAAGGAATCGGTCAA T | Characterization of the clones |
| Hygro rev SEQ ID NO: 27 | TGCTGCTCCATACAAGCCA A | Characterization of the clones |
| Cas9 fwd SEQ ID NO: 28 | CCCTGCTGTTCGACAGCGG CGAAACAGCCGAGG | Characterization of the clones |
| Cas9 rev SEQ ID NO: 29 | GGCATCCTCGGCCAGGTCG AAGTTGCTCTTGAAGTTGG G | Characterization of the clones |
| eGFP_2 fwd SEQ ID NO: 30 | GACCTACGGCGTGCAGTGC TTCAGCAGATACCC | Characterization of the clones |
| eGFP_2 rev SEQ ID NO: 31 | GTTCACTTTGATGCCGTTTT TCTGCTTGTCGGCC | Characterization of the clones |

### Cell lines, cell culture media and additives:

All experiments were performed with HEK293A cells (Quantum Biotechnologies Inc.).

| **Material** | **Manufacturer** |
|---|---|
| Accutase | Sigma |
| Fetal calf serum (FCS) | Gibco |
| Hygromycin B (50 mg/mL) | Gibco |
| L-glutamine (200 mM) | Gibco |
| PBS (1×) | PAN |
| Penicillin-streptomycin (10,000 U/mL) | Gibco |
| Puromycin dihydrochloride (10 mg/mL) | Gibco |
| RPMI 1640 | PAN |

### Antibody:

The antibodies were stored according to the manufacturer's instructions at 4 °C or - 20 °C.

| **Antibody** | **Species/ Clonalit**y | **Manufactur er** | **Dilution** | **Use** |
|---|---|---|---|---|
| α-beta actin HRP (Ab4990) | Mouse/ monoclonal | Abcam | 1:15,000 | Western blot |
| α-Cas9 (Ab 189380) | Rabbit/ monoclonal | Abcam | 1:15,000 | Western blot |
| α-GFP-Alexa Fluorine 647 (A-31852) | Rabbit/ polyclonal | Life Technologie s | 1:1000 | Imaging analyses |
| α-GFP-HRP (Ab 190584) | Rabbit/ monoclonal | Abcam | 1:10,000 | Western blot |
| α-human CD4-APC (#17-0049-41) | Mouse/ monoclonal | eBioscience | 1:50 | eGFP *Knock-in* V3 *Pool Sort* |
| α-Rabbit IgG-HIZP (#1706515) | Goat/ polyclonal | Bio-Rad | 1:3000 | Western blot |
| α-TUBA1B (Ab 108629) | Rabbit/ monoclonal | Abcam | 1:15,000 | Western blot |

### Kits:

| **Kit system** | **Manufacturer** | **Use** |
|---|---|---|
| Cell Lysis Kit | Roche | Production of cell lysates |
| CellTiter Glo Assay Kit | Promega | Killing curve assay |
| Seamless Cloning and Assembly Kit | Invitrogen | Cloning donor |
| Genomic Cleavage Detection Kit | Invitrogen | Surveyor assay |
| KOD Hot Start DNA polymerase | Merck | cloning |
| NucleoBond Xtra Midi Plus Kit | Macherey-Nagel | Plasmid |
| One Shot Top10 Chemically Competent *E. coli* | Invitrogen | cloning |
| Pierce BCA Protein Assay Kit | Invitrogen | BCA assay |
| QIAprep Spin Miniprep Kit | Qiagen | plasmid |
| QIAquick Gel Extraction Kit | Qiagen | cloning |
| QIAquick PCR Purification Kit | Qiagen | Cloning, sequencing |
| RevertAid First Strand cDNA Synthesis Kit | ThermoFisher | cDNA synthesis |
| RNeasy Micro Kit | Qiagen | RNA isolation |

### Methods:

### 1. Molecular biological work

### 1.1. Hybridization of Synthetic Oligonucleotides

The DNA sequences coding for the gRNA in the all-in-one pCas-Guide EF1α-CD4 plasmid from Origene were ordered as synthetic oligonucleotides. To be able to clone them into the vector, first the complementary single strands had to be hybridized. They were designed such that each 5'-overhang was complementary to a 3'-overhang of the linearized vector. This achieves precise ligation in the desired orientation.

A reaction for hybridization was set up as follows:

| | |
|---|---|
| Annealing buffer (10×) | 2 µL |
| Forward oligo (100 µM) | 1 µL |
| Reverse oligo (100 µM) | 1 µL |
| Water | 16 µL |
| Total | 20 µL |

The mixture was mixed well and incubated in the thermocycler according to the following program:

| | |
|---|---|
| 95 °C | 5 min |
| 95 °C - 20 °C | -2 °C/min |
| 20 °C | 60 min |
| 4 °C | ∞ |

The samples were diluted 1: 10 with water and stored at -20 °C until further use.

### 1.2. Polymerase Chain Reaction

The polymerase chain reaction, PCR for short, was performed with the KOD Hot Start DNA Polymerase Kit from Novagen. Among other things, the method was used to generate the homologous sequences and the eGFP fragment for the cloning of the donor plasmid, and later to characterize the selected clones. To generate fragments of the donor plasmid, the PCR primers were designed specifically to generate short 15 bp homologous sequences at the ends of adjacent fragments. Using this microhomology, Invitrogen's Seamless Cloning and Assembly Kit was later used to ensure accurate ligation in the correct order and orientation.

In addition, the forward primer for the generation of the 3' homologue arm D_3' HA fwd contained several silent point mutations. These were located in the sequence region bound by the crRNAs used. To protect the donor plasmid from transfection in front of the Cas9 nuclease, the crRNA binding site must be mutated. The introduction of a silent mutation in the PAM sequence was not possible due to the reading frame, and would have caused a change in the protein sequence. Therefore, all possible bases which would not later produce any change in the protein sequence were exchanged.

A PCR reaction consisted of the following approach:

| **DNA template** | **50 ng (plasmid DNA)** |
|---|---|
| | **100 ng (genomic DNA)** |
| | **1 µL (cDNA)** |
| Forward primer (4 µM) | 5 µL |
| Reverse primer (4 µM) | 5 µL |
| Reaction buffer (10×) | 5 µL |
| MgSO₄ (25 mM) | 3 µL |
| dNTP mix (2 mM each) | 5 µL |
| KOD polymerase | 1 µL |
| water | x µL |
| **Total** | **50 µL** |

The PCR program differs depending on the type of template, the length of the PCR product and the nature of the primers used, such as the GC content and the melting temperature. By default, the following PCR programs were used:

| **PCR on plasmids and cDNA** | | | | **PCR for genomic DNA** | | | |
|---|---|---|---|---|---|---|---|
| 1 cycle | 95 °C | | 5 min | 1 cycle | 95 °C | | 5 min |
| 30 cycles | 95 °C | | 15 s | 40 cycles | 95 °C | | 20 s |
| 65 °C-70 °C (depending on primers) | | 20 s | | 65 °C-70 °C (depending on primers) | | 20 s | |
| 72 °C | | 30 s each kb (depending on product length) | | 72 °C | | 30 s each kb (depending on product length) | |
| 1 cycle | 72 °C | | 5 min | 1 cycle | 72 °C | | 5 min |
| 1 cycle | 4 °C | | ∞ | 1 cycle | 4 °C | | ∞ |

### 1.3. Restriction digestion

One component of a cloning is the restriction digestion with sequence-specific restriction endonucleases. On the one hand, DNA fragments to be cloned are prepared. On the other hand, the method can be used as a restriction analysis in which a cloned plasmid is examined for its correct assembly. Optimized high-fidelity restriction enzymes from NEB were preferably used.

In general, a restriction mixture consisted of the following components:

| | |
|---|---|
| DNA (e.g., plasmids, PCR product) | x µL |
| Restriction enzyme(s) | 0.25 µL/µg DNA |
| CutSmart buffer (10×) | 3 µL |
| Water | y µL |
| Total | 30 µL |

The mixture was mixed well and incubated for at least one hour at 37 °C in the thermoblock. Subsequently, the temperature was raised to 70 °C for 10 minutes in order to inactivate the restriction enzymes.

In this way, the donor plasmid with the hygromycin B resistance cassette was also linearized with the endonuclease NotI-HF for transfection. 1 µL of shrimp alkaline phosphatase (rSAP) was then directly added to the restriction mixture and incubated again at 37 °C for one hour. This dephosphorylated the vector ends, preventing random religation of the plasmid.

When using restriction enzymes that show maximum activity at different temperatures or in different buffers, the digestion had to be done in two temperature steps or a step for rebuffering should be incorporated.

### 1.4. Agarose gel electrophoresis and gel extraction

By means of gel electrophoresis, mixtures of DNA fragments in the agarose gel can be separated according to their size. The samples are added to this with 6× loading buffer and applied to a 1% agarose gel with 0.5 µg/mL ethidium bromide. After applying the samples, a voltage of 100 V was applied for one hour. As a result, the negatively charged DNA travels through the gel towards the anode. The size and conformation of the DNA is crucial for the running behavior in the gel. Gel electrophoresis is the standard for preparative restriction digestion or PCR. In the restriction digest of a plasmid, the desired DNA fragment can thus be separated from the remaining part of the plasmid and the specific DNA band can be excised from the gel.

In PCR, optimally only exactly one specific fragment should be amplified. Gel electrophoresis reveals how pure the PCR is and whether non-specific by-products have been formed. Again, the specific DNA band can then be cut out of the gel at the expected height.

To extract the desired DNA from the excised agarose gel piece, the QIAquick Gel Extraction Kit from Qiagen was used according to the accompanying protocol. Briefly, the gel piece was dissolved in a binding buffer at 50 °C, treated with isopropanol and placed on a column. The DNA binds to the silica membrane of the column and was eluted from the column after a short washing step with elution buffer. Thus, among others, salts, enzymes or other impurities, which may later interfere with applications such as a sequencing reaction, can be removed from the reaction mixture. The purified DNA was analyzed on NanoDrop2000 and stored at -20 °C.

### 1.5. Ligation and transformation in E. coli

In ligation, DNA fragments with complementary ends are linked together using an ATP-dependent DNA ligase. For the ligation reaction, T4 DNA ligase from NEB was used. The plasmid backbone and insertion fragment were used in a molar ratio of 1:10 in the ligation batch.

The general approach was:

| | |
|---|---|
| DNA backbone | x µL |
| DNA insert | y µL |
| Ligation buffer (10×) | 2 µL |
| T4 DNA ligase | 1 µL |
| Water | z µL |
| total | 20 µL |

The mixture was mixed well and incubated for at least 1 h in a thermocycler at 16 °C. Subsequently, the ligase was inactivated for 10 min. at 70 °C.

An exception was the ligation of the fragments for the donor plasmids. These fragments were assembled using Invitrogen's Seamless Cloning and Assembly Kit according to the accompanying protocol. As a result of the primers used, adjacent fragments had a short microhomology of 15 bp over which the fragments were ligated in the correct order and orientation.

The resulting plasmid was then introduced for propagation into chemically competent *E. coli.* The transformation was performed with Chemically Competent OneShot Top10 *E. coli* from Invitrogen. For this purpose, the bacteria stored at -80 °C were thawed on ice, mixed with 2 µL of the ligation mixture and incubated on ice for at least 30 min. Then the heat shock occurred, in which the plasmid is introduced into the competent cells. For this purpose, the cells were incubated for 30 seconds in a water bath at 42 °C and then for 2 min. on ice. After addition of 200 µL SOC medium, the cells were incubated for one hour at 37 °C and 700 rpm in a thermo shaker. 50 µL of the transformation mixture was plated on LB agar plates. Depending on the resistance cassette, the agar plates contained on the incorporated plasmid 100 µg/mL ampicillin or 50 µg/mL kanamycin. Overnight, the plates incubated at 37 °C, allowing only bacterial colonies to grow that had received the desired plasmid.

### 1.6. Plasmid Preparation

The plasmid preparation is a method for the isolation of cloned plasmid DNA from transformed *E. coli.* For this, individual colonies were picked from the agar plate, transferred to LB medium and cultured overnight at 37 °C and 200 rpm. Depending on the desired yield different sized approaches were made with LB medium. A distinction was made between mini preparations with 2 mL, medium preparations with 150 mL, maxi preparations with 400 mL and giga preparations with up to 2.5 L.

All plasmid preparations were performed with kits from Qiagen and Macherey-Nagel according to the accompanying protocol. In principle, the overnight culture was centrifuged and the bacterial pellet resuspended with RNase-containing buffer. The cells were lysed with lysis buffer for 5 min. and placed on a column. The DNA bound to the silica membrane of the column and was eluted after a washing step with elution buffer. In the medium and maxi preparations, the DNA in the eluate was again precipitated with isopropanol and centrifuged for one hour at 4,500 rpm. After a washing step with 70 % ethanol, the DNA pellet was dried for 15 minutes at room temperature and then re-dissolved with 500 µL TRIS buffer. Analysis of the sample on the NanoDrop2000 revealed the concentration and purity of the DNA. The DNA thus obtained was stored at -20 °C and later used for HEK293A transfection.

### 2. Cultivation of human cells

The HEK293A cells were cultivated in the incubator at 37 °C and 5% CO₂. The complete medium consisted of RPMI 1640 with 10% FCS and 2 mM L-glutamine. For continuous cultivation, the cells were split twice weekly, reaching between 70 % to 90 % confluence. For this, the medium was aspirated, the cells washed with PBS and detached with Accutase. After resuspension with fresh medium, 1/10 of the cell suspension was transferred to a new T75 cell culture flask. When 25 passages were reached, the continuous culture was discarded and fresh cells from the internal cell bank were thawed.

The generated clones were cultured in the same complete medium as the wild-type cells and split 1:5 twice a week. From each clone at least three cryotubes with 3×10⁶ cells were frozen as backup. For this purpose, the cells were centrifuged for 5 min at 300 g and resuspended in 1 mL of freezing medium consisting of FCS with 7.5% DMSO. The cells were transferred to a cryotube and frozen in a freezer box at -80 °C overnight. The next day, the frozen cells were transferred to the nitrogen tank for long-term storage.

### 3. Transfection by Lipofectamine

In order to achieve a specific knock-in in the cells, various plasmid DNA and partially synthetically produced RNA had to be introduced into the cells. Depending on the sample to be introduced, the two transfection reagents DharmaFECT Duo from Dharmacon and TurboFectin 8.0 from Origene were used.

The transfection was carried out for the knock-in experiments in a 6-well format, wherein the optimization of the transfection protocol was performed in a 96-well format. The cells were seeded the day before so that they reached confluency of about 70-80 % at the time of transfection. Generally, for transfection with lipofectamines, the DNA and the transfection reagent were mixed together in serum-reduced Opti-MEM. The batches had to incubate for some time at room temperature to form complexes of lipofectamines and the nucleic acids. Subsequently, the transfection mixtures were added dropwise to the cells.

Depending on the transfection reagent, the following optimized protocol was used in a 6-well format:

| **Transfection reagent** | **DharmaFECT duo** | **TurboFectin 8.0** | |
|---|---|---|---|
| Transfected sample | Plasmid DNA and synthetic RNAs | Plasmid DNA | |
| Transfected DNA/RNA amount per *well* in a 6*-well* format | - a total of 6.5 µg of plasmid DNA | - a total of 6.5 µg of plasmid DNA | |
| | - 25 nM each of synthetic RNA | | |
| Transfection preparation | - 20 µL DharmaFECT Duo diluted in 230 µL OptiMEM -> gives a working solution of 80 µg/mL | - Dilute 19.5 µL TurboFectin 8.0 in | |
| | | Opti-MEM to 100 µL | |
| | - Donor plasmid, Cas9-PuroR plasmid and synthetic RNAs in | - Incubate for 5 min at room temperature | |

| | Opti-MEM diluted to 250 µL | | Addition of pCas9-Guide-EF1α-CD4 plasmid and the donor plasmid |
|---|---|---|---|
| | - both approaches mixed well | | |
| | | | - mixed well |
| Incubation of the batches | | 20 min at room temperature | |
| Transfection | | Add the preparation drop-by-drop to the cells | |

### 4. CellTiter-Glo assay

The CellTiter-Glo assay was used to determine the number of viable cells in a sample. The method is based on the quantification of the ATP amount as an indicator of metabolically active cells. The CellTiter-Glo Luminescent Cell Viability Assay Kit from Promega was used for the experiments. The CellTiter-Glo reagent was freshly prepared before each experiment. For this purpose, the substrate, which contains *inter alia* luciferin and a luciferase, was dissolved in the accompanying buffer. After direct addition to the medium of the cells, lysis of the cells occurs first. Viable cells produce ATP in their mitochondria, which is thereby released. In the presence of ATP, the luciferin is converted by the UltraGlo r-luciferase. This produces a stable luminescence signal which is proportional to the amount of ATP present and thus to the number of viable cells.

The assay was used to optimize transfection and to titrate the optimal antibiotic concentrations of hygromycin B and puromycin for post-transfection selection. In all assays, cells were seeded in a black flat bottom 96-well plate with a transparent bottom. When titrating the antibiotic concentrations, 7,500 cells were seeded per well. The next day, the cells were treated with various concentrations of the antibiotic. After 48 hours, the viability of the cells was determined by the assay in which 100 µL of the CellTiter-Glo reagent was added to each well. Subsequently, the plate was shaken on a plate shaker for 2 min. at 200 rpm. After incubation in the dark for 10 minutes, the luminescence signal was measured on the luminometer.

### 5. Flow cytometry

### 5.1. FACS Sorting

FACS stands for Fluorescence Activated Cell Sorting and is a special form of flow cytometry. With FACS sorting, the desired cells can be sorted from a cell suspension. A distinction is made between the storage of the desired cells as a cell pool in a Falcon or as individual cells in a 96-well plate. The experiments were carried out on a FACS Aria III sorter from BD.

Using FACS sorting, the transfected and partially already selected cells were sorted. These were harvested, washed with PBS and adjusted to a cell number of 1×10⁶ cells/mL in FACS buffer. After the desired cell population had been gated and all settings made on the device for sorting, the selected cells could be discarded. The single cell deposit was performed in 96-well format. The plates were stored with 200 µL of medium per well at 37 °C in order to temper the medium and to keep the cell's stress as low as possible. Each knock-in trial involved deposition in between 20 and 24 plates. The cells then needed about 3 weeks in the incubator at 37 °C and 5% CO₂ for growth. All plates were analyzed microscopically to determine the growth rate and the clones were then examined for eGFP expression.

In the knock-in V3, the pCas9-Guide-EF1α-CD4 plasmid contains a CD4 cassette as a selection marker. To select the CD4-positive cells, the cells were harvested 48 hours after transfection and washed twice with PBS. Next, the cells were resuspended in 250 µL FACS buffer and 5 µL α-CD4-Alexa 647 antibody added. This corresponded to a final antibody concentration of 1.5 µg/mL. After one hour of incubation on ice in the dark, the cells were washed three times with FACS buffer and then measured. For pool sorting, approximately 1.5×10⁵ CD4-positive cells per sample were sorted into a 15 mL Falcon in which 5 mL of complete medium had already been placed. Subsequently, the cells were centrifuged for 5 min. at 300 g, resuspended in 3 mL fresh complete medium and seeded into a 6-well plate. After 24 hours the cells were again adherent and could be selected with hygromycin B on the donor plasmid. Once the selected cells had matured, the single-cell deposition in the 96-well format was again carried out.

### 5.2. Analytical flow cytometry

Analytical flow cytometry is based on the same functional principle as FACS sorting. The only difference is that the cells cannot be sorted, but can only be examined for size, granularity and possibly fluorescence properties.

The methodology was used primarily for the screening of the generated clones. For this purpose, the medium was completely removed from the 96-well plates, the cells were detached with 25 µL Accutase and resuspended with 40 µL FACS buffer. 40 µL of cell suspension was transferred to a 96-well conical-bottomed plate and measured directly on the FACS Canto II from BD. The eGFP signals of the clones were always referenced with HEK293A wild-type cells. The evaluation was done with the FlowJo v10.0.7 software. Potentially positive clones with a distinct eGFP signal were expanded and further characterized.

### 6. Total protein extraction and BCA assay

In order to verify the eGFP knock-in also at the protein level, the total protein had to be extracted from the cells. For this purpose, 1×10⁶ cells were harvested, centrifuged for 5 minutes at 300 g and washed with ice-cold PBS. After a repeated centrifugation step, the supernatant was completely removed from the cells. The cell pellet was resuspended in 60 µL freshly prepared RIPA buffer and incubated on ice for at least 30 minutes. Then the lysates were centrifuged for 15 min. at 14,000 rpm and 4 °C to remove cell debris. The supernatant was transferred to a new reaction vessel and stored at -20 °C.

Using a bicinchoninic acid assay, BCA assay for short, the protein concentration in the lysates was quantified. The assay relies on protein-dependent reduction of divalent copper ions to monovalent copper ions, which form a color complex with BCA. The color change resulting from the reaction is directly proportional to the protein concentration. The assay was performed using the ThermoFisher Pierce BCA Protein Assay Kit according to the associated protocol. A standard series with protein concentrations between 2 mg/mL and 125 µg/mL was prepared from a 2 mg/mL BSA stock solution. In a 96-well flat-bottomed plate 10 µL BSA standard or 10 µL diluted lysate were submitted, with a duplicate determination made in each case. Then 200 µL of BCA solution consisting of BCA Reagent A and B were added to each well in the ratio 50: 1. The plate was shaken for 30 seconds on the plate shaker and then incubated for 30 min. at 37 °C. The resulting color change was measured on the photometer at a wavelength of 562 nm. The values of the standard series were used to create a calibration line, from whose equation of function the protein concentrations in the lysates could be calculated.

### 7. SDS page and Western Blot

To verify the eGFP knock-in at the protein level, the resulting protein lysates were used to make an SDS-Page followed by a Western blot.

In each case, 15 µg of total protein were adjusted to 10.8 µE with PBS and 6 µL of 2.8-fold LDS loading buffer was added. The samples were then boiled for 10 min. at 95 °C and centrifuged for 2 min. at 12,000 rpm. The samples were applied to Invitrogen's NuPAGE 4-12% Bis-Tris gel and were run at 220 V and 120 mA for 50 minutes. In the wet blot, the proteins are blotted for one hour at 30 V and 220 mA onto a nitrocellulose membrane. As a control for a successful protein transfer, the colored protein standard was used. The membrane was incubated for at least two hours at room temperature in 5% block solution on the shaker to block the epitopes of the proteins and reduce later non-specific antibody binding. The primary antibody was diluted in 5% blocking solution and incubated overnight at 4 °C on the rocking shaker. The next day, the membrane was washed three times with TBST for 15 min. to remove unbound primary antibody. Subsequently, the membrane was incubated with the secondary antibody diluted in 5% block solution for two hours at room temperature on the shaker. Again, TBT had to be washed three times for 15 min. to remove unbound secondary antibody. To develop the blot, the Lumi-Light Western Blotting Substrate Kit from Roche was used. The luminol enhancer solution and the peroxidase solution were mixed in the same ratio, added to the membrane and incubated for 3 min. in the dark. The secondary antibody was coupled with a horseradish peroxidase (HRP), which catalyzes the oxidation of luminol. The resulting luminescence signals could be detected on the Lumi imager. The exposure time varied depending on the band intensities. After development, the membrane was incubated for 15 minutes at room temperature in Restore PLUS Western Blot Stripping Buffer, removing bound antibody from the membrane. Next, the membrane was washed three times with TBST for 10 minutes and incubated again for at least two hours at room temperature with 5% block solution. The membrane was then cut just below the 50 kDa marker band. The upper part of the membrane was incubated with an α-GFP HRP-coupled antibody diluted in 5% block solution overnight at 4 °C. The lower part of the membrane was stored overnight in 5% block solution and incubated with an α-beta-actin HRP-coupled antibody for 15 min. at room temperature the next day. Both membrane parts were washed three times with TBST for 15 min. Since these two primary antibodies are HRP-coupled, the membrane could be developed without further incubation with a secondary antibody analogous to the previous day.

### 8. Confocal microscopic analysis in the Operetta system

By confocal microscopic analysis, the tubulin cytoskeleton in the potential clones was examined. The experiments were performed on the Operetta CLS High-Content Imaging System by Perkin Elmer. With this system, a large number of clones could be screened automatically in a 96-well format over a short time. Since the endogenous eGFP signal for resolution in the Operetta system was quite weak, the cells were still stained for signal amplification using an α-GFP-Alexa Fluor 647 antibody.

For the screening, 1.2×10⁴ cells were seeded in 96-well plates. The plates were black flat bottom plates with a transparent bottom, which, in addition to their low autofluorescence, also prevented the entry of stray light into adjacent wells. The next day, the cells were washed with PBS and fixed with ice-cold methanol for 5 min. The α-GFP Alexa Fluor 647 antibody used was diluted 1: 1,000 in Goat Serum Dilution Buffer (GSDB) to a final concentration of 1 µg/mL. The buffer contained inter alia Triton-X-100, which leads to the permeabilization of the cell membranes and thus allows the penetration of the antibody into the cells. After two hours of incubation at room temperature in the dark, the antibody was removed from the cells. For the staining of the cell nuclei, Hoechst 33342 was diluted 1: 10,000 in PBS and incubated for 10 min. at room temperature in the dark. Several washes were done to remove free antibody and unbound Hoechst dye from the cells. First, it was washed twice with a high-salt sodium phosphate buffer for 5 min. Subsequently, the washing steps were repeated analogously with a low-salt sodium phosphate buffer, which promoted a transport of free antibody out of the cells. For fluorescence imaging in the Operetta system, the cells were overlaid after washing with 200 µL PBS.

All the necessary settings in the Operetta system, had to be made first. In addition to the choice of the objective and general settings for the plate geometry, this also included the selection of the required fluorescence channels with the respective exposure times and sharpening levels. The device ran all selected pixels in each marked well and took one picture individually for each channel. These were processed and evaluated with the associated Harmony Imaging and Analysis software.

## Claims

1. A method for producing or providing cells expressing a fusion protein of a marker protein and a cell endogenous target protein from the endogenous gene locus of the target protein, wherein the method comprises the following steps:
a) transfecting the cells
i) with a Cas9-encoding plasmid comprising a nucleic acid, which confers resistance to a first selection reagent,
ii) with a circular donor plasmid comprising
a) a first nucleic acid, which confers resistance to a second selection reagent, and
b) a second nucleic acid, which is encoding the marker protein,
whereby the second nucleic acid is flanked 3' and 5' by nucleic acids homologous to the integration site in the cell and whereby one of the flanking homologous nucleic acids is homologous to the terminal coding sequence of the target protein,
iii) with a crRNA,
and
iv) with a tracrRNA,
b) culturing the cells in the presence of the first and the second selection reagents,
and
c) selecting cells that perform cell division under the conditions of step b) and thereby providing cells that express the fusion protein of a marker protein and a cell-endogenous target protein.

2. The method of claim 1, wherein the cells in step a) are transfected simultaneously with all the plasmids and nucleic acids.

3. The method according to any one of claims 1 to 2, wherein in step c) cells are selected, which perform cell division and in which the fusion protein has been detected.

4. The method according to any one of claims 1 to 3, wherein steps b) and c) are the following steps:
b)
1) culturing the cells in the presence of only the first selection reagent or only the second selection reagent,
2) selecting cells that perform cell division under the conditions of step 1),
3) culturing the cells selected in step 2) in the presence of the selection reagent which was not used in step 1),
c) selecting cells, which under the conditions of step b) 3) perform cell division and in which the fusion protein has been detected, and thereby providing cells that express the fusion protein of a marker protein and a cell endogenous target protein from the endogenous gene locus of the target protein.

5. The method according to any one of claims 1 to 4, wherein the selection reagents are puromycin and hygromycin B.

6. The method according to claim 5, wherein in step b) or in steps b) 1) and b) 3) the final hygromycin B concentration is 50 µg/mL and the final puromycin concentration is 2 µg/mL.

7. The method according to any one of claims 1 to 6, wherein in the presence of multiple possible sites of insertion for the marker protein encoding nucleic acid, the nucleic acid encoding the marker protein is inserted at the site containing more and/or more specific gDNA binding sites.

8. The method according to any one of claims 1 to 7, wherein the cells are mammalian cells.

9. The method according to any one of claims 1 to 8, wherein
i) the marker protein is inserted N-terminally to the target protein,
ii) the nucleic acid encoding the marker protein is inserted in the endogenous gene locus so that it is directly before (3' to) the first codon of the target protein in the mRNA of the fusion protein,
and
iii) the 3'-flanking nucleic acid contains the start codon of the nucleic acid encoding the target protein or/and the 5'-flanking nucleic acid is homologous to the N-terminus of the target protein.

10. The method according to any one of claims 1 to 9, wherein the nucleic acid encoding the marker protein does not include a start codon.

11. The method according to any one of claims 1 to 10, wherein the marker protein is a fluorescent marker protein.

12. The method according to claim 11, wherein the fluorescent marker protein is green fluorescent protein (GFP).

13. The method according to any one of claims 1 to 12, wherein the 3'- and 5'-flanking homologous nucleic acids have a size of about 1000 nucleotides.

## Patentansprüche

1. Verfahren zum Produzieren oder Bereitstellen von Zellen, die ein Fusionsprotein eines Markerproteins und eines zellendogenen Zielproteins aus dem endogenen Genort des Zielproteins exprimieren, wobei das Verfahren die folgenden Schritte umfasst:
a) Transfizieren der Zellen
i) mit einem Cas9-kodierenden Plasmid, das eine Nukleinsäure umfasst, die Resistenz gegen ein erstes Auswahlreagens verleiht,
ii) mit einem ringförmigen Donorplasmid, umfassend
a) eine erste Nukleinsäure, die Resistenz gegen ein zweites Auswahlreagens verleiht, und
b) eine zweite Nukleinsäure, die für das Markerprotein kodiert,
wodurch die zweite Nukleinsäure 3' und 5' von Nukleinsäuren flankiert wird, die homolog sind zu der Integrationsstelle in der Zelle, und wodurch eine der flankierenden homologen Nukleinsäuren homolog ist zu der terminalen kodierenden Sequenz des Zielproteins,
iii) mit einer crRNA
und
iv) mit einer tracrRNA,
b) Kultivieren der Zellen in der Gegenwart des ersten und des zweiten Auswahlreagens
und
c) Auswählen von Zellen, die unter den Bedingungen von Schritt b) Zellteilung vornehmen, und dadurch Bereitstellen von Zellen, die das Fusionsprotein eines Markerproteins und eines zellendogenen Zielproteins exprimieren.

2. Verfahren nach Anspruch 1, wobei die Zellen in Schritt a) gleichzeitig mit allen Plasmiden und Nukleinsäuren transfiziert werden.

3. Verfahren nach einem der Ansprüche 1 bis 2, wobei in Schritt c) die Zellen ausgewählt werden, die Zellteilung vornehmen und in denen das Fusionsprotein nachgewiesen worden ist.

4. Verfahren nach einem der Ansprüche 1 bis 3, wobei die Schritte b) und c) die folgenden Schritte sind:
b)
1) Kultivieren der Zellen in der Gegenwart von nur dem ersten Auswahlreagens oder nur dem zweiten Auswahlreagens,
2) Auswählen von Zellen, die unter den Bedingungen von Schritt 1) Zellteilung vornehmen,
3) Kultivieren der in Schritt 2) ausgewählten Zellen in der Gegenwart des Auswahlreagens, das in Schritt 1) nicht verwendet wurde,
c) Auswählen von Zellen, die unter den Bedingungen von Schritt b) 3) Zellteilung vornehmen und in denen das Fusionsprotein nachgewiesen worden ist, und dadurch Bereitstellen von Zellen, die das Fusionsprotein eines Markerproteins und eines zellendogenen Zielproteins aus dem endogenen Genort des Zielproteins exprimieren.

5. Verfahren nach einem der Ansprüche 1 bis 4, wobei die Auswahlreagenzien Puromycin und Hygromycin B sind.

6. Verfahren nach Anspruch 5, wobei in Schritt b) oder in den Schritten b) 1) und b) 3) die Hygromycin-B-Endkonzentration 50 µg/ml beträgt und die Puromycin-Endkonzentration 2 µg/ml beträgt.

7. Verfahren nach einem der Ansprüche 1 bis 6, wobei in der Gegenwart mehrerer möglicher Einfügungsstellen für die für das Markerprotein kodierende Nukleinsäure die Nukleinsäure, die für das Markerprotein kodiert, an der Stelle eingefügt wird, die mehrere und/oder spezifischere gDNA-Bindungsstellen enthält.

8. Verfahren nach einem der Ansprüche 1 bis 7, wobei die Zellen Säugerzellen sind.

9. Verfahren nach einem der Ansprüche 1 bis 8, wobei
i) das Markerprotein N-terminal in Bezug auf das Zielprotein eingefügt wird,
ii) die Nukleinsäure, die für das Markerprotein kodiert, in den endogenen Genort eingefügt wird, sodass sie sich direkt vor (3' zu) dem ersten Codon des Zielproteins in der mRNA des Fusionsproteins befindet,
und
iii) die 3'-flankierende Nukleinsäure das Startcodon der Nukleinsäure enthält, die für das Zielprotein kodiert, oder/und die 5'-flankierende Nukleinsäure homolog ist zu dem N-Terminus des Zielproteins.

10. Verfahren nach einem der Ansprüche 1 bis 9, wobei die Nukleinsäure, die für das Markerprotein kodiert, kein Startcodon einschließt.

11. Verfahren nach einem der Ansprüche 1 bis 10, wobei das Markerprotein ein fluoreszierendes Markerprotein ist.

12. Verfahren nach Anspruch 11, wobei das fluoreszierende Markerprotein ein grün fluoreszierendes Protein (GFP) ist.

13. Verfahren nach einem der Ansprüche 1 bis 12, wobei die 3'- und 5'-flankierenden homologen Nukleinsäuren eine Größe von etwa 1.000 Nukleotiden haben.

## Revendications

1. Procédé de production ou de fourniture de cellules exprimant une protéine de fusion d'une protéine marqueur et d'une protéine cible endogène de cellule à partir du locus du gène endogène de la protéine cible, dans lequel le procédé comprend les étapes suivantes :
a) transfection des cellules
i) avec un plasmide codant pour Cas9 comprenant un acide nucléique, qui confère une résistance à un premier réactif de sélection,
ii) avec un plasmide donneur circulaire comprenant
a) un premier acide nucléique, qui confère une résistance à un second réactif de sélection, et
b) un second acide nucléique, qui code pour la protéine marqueur,
moyennant quoi le second acide nucléique est flanqué en 3' et 5' par des acides nucléiques homologues au site d'intégration dans la cellule et moyennant quoi l'un des acides nucléiques homologues de flanquage est homologue à la séquence codante terminale de la protéine cible,
iii) avec un ARNcr,
et
iv) avec un ARNtracr,
b) culture des cellules en présence des premier et second réactifs de sélection,
et
c) sélection des cellules qui réalisent une division cellulaire dans les conditions de l'étape b) et fourniture ainsi de cellules qui expriment la protéine de fusion d'une protéine marqueur et d'une protéine cible endogène de cellule.

2. Procédé selon la revendication 1, dans lequel les cellules de l'étape a) sont transfectées simultanément avec tous les plasmides et acides nucléiques.

3. Procédé selon l'une quelconque des revendications 1 à 2, dans lequel à l'étape c) des cellules sont sélectionnées, lesquelles réalisent une division cellulaire et dans lesquelles la protéine de fusion a été détectée.

4. Procédé selon l'une quelconque des revendications 1 à 3, dans lequel les étapes b) et c) sont les étapes suivantes :
b)
1) culture des cellules en présence uniquement du premier réactif de sélection ou uniquement du second réactif de sélection,
2) sélection des cellules qui réalisent une division cellulaire dans les conditions de l'étape 1),
3) culture des cellules sélectionnées à l'étape 2) en présence du réactif de sélection qui n'a pas été utilisé à l'étape 1),
c) sélection des cellules, qui dans les conditions de l'étape b) 3) réalisent une division cellulaire et dans lesquelles la protéine de fusion a été détectée, et fourniture ainsi de cellules qui expriment la protéine de fusion d'une protéine marqueur et d'une protéine cible endogène de cellule à partir du locus du gène endogène de la protéine cible.

5. Procédé selon l'une quelconque des revendications 1 à 4, dans lequel les réactifs de sélection sont la puromycine et l'hygromycine B.

6. Procédé selon la revendication 5, dans lequel à l'étape b) ou aux étapes b) 1) et b) 3) la concentration finale en hygromycine B est de 50 µg/mL et la concentration finale en puromycine est de 2 µg/mL.

7. Procédé selon l'une quelconque des revendications 1 à 6, dans lequel en présence de multiples sites d'insertion possibles pour l'acide nucléique codant pour la protéine marqueur, l'acide nucléique codant pour la protéine marqueur est inséré au niveau du site contenant des sites de liaison à l'ADNg supplémentaires et/ou plus spécifiques.

8. Procédé selon l'une quelconque des revendications 1 à 7, dans lequel les cellules sont des cellules de mammifère.

9. Procédé selon l'une quelconque des revendications 1 à 8, dans lequel
i) la protéine marqueur est insérée de manière N-terminale à la protéine cible,
ii) l'acide nucléique codant pour la protéine marqueur est inséré dans le locus du gène endogène de sorte qu'il est immédiatement avant (en 3' par rapport au) le premier codon de la protéine cible dans l'ARNm de la protéine de fusion,
et
iii) l'acide nucléique de flanquage en 3' contient le codon d'initiation de l'acide nucléique codant pour la protéine cible ou/et l'acide nucléique de flanquage en 5' est homologue à l'extrémité N-terminale de la protéine cible.

10. Procédé selon l'une quelconque des revendications 1 à 9, dans lequel l'acide nucléique codant pour la protéine marqueur ne comprend pas de codon d'initiation.

11. Procédé selon l'une quelconque des revendications 1 à 10, dans lequel la protéine marqueur est une protéine marqueur fluorescente.

12. Procédé selon la revendication 11, dans lequel la protéine marqueur fluorescente est une protéine fluorescente verte (GFP).

13. Procédé selon l'une quelconque des revendications 1 à 12, dans lequel les acides nucléiques homologues de flanquage en 3' et 5' ont une taille d'environ 1000 nucléotides.
